(19) Europäisches Patentamt
European Patent Office
Office européen des brevets

(11) Publication number: **0 402 092**
**A2**

(12) **EUROPEAN PATENT APPLICATION**

(21) Application number: **90306088.7**

(22) Date of filing: **05.06.90**

(51) Int. Cl.5: **C12N 15/56, C12N 9/28, C12N 9/44, C12P 19/14, C12P 19/16, //(C12N15/56, C12R1:145)**

(30) Priority: **05.06.89 US 361368**

(43) Date of publication of application: **12.12.90 Bulletin 90/50**

(84) Designated Contracting States: **AT BE CH DE DK ES FR GB GR IT LI LU NL SE**

(71) Applicant: **Alko Ltd.**
**P.O. Box 350**
**SF-00101 Helsinki(FI)**

(72) Inventor: **Melasniemi, Hannes**
**Putousrinne 1 F 60**
**SF-01600 Vantaa(FI)**
Inventor: **Paloheimo, Marja Tuulikki**
**Oksasenkatu 7 A 24**
**SF-00100 Helsinki(FI)**

(74) Representative: **Sheard, Andrew Gregory et al**
**Kilburn & Strode 30, John Street**
**London WC1N 2DD(GB)**

(54) **Alpha-amylase-pullulanase enzyme.**

(57) A gene from Clostridium thermohydrosulphuricum DSM 3783 encoding a thermostable alpha-amylase-pullulanase enzyme was cloned encoding in Escherichia coli as a 7 kb insert using a lambda-vector. For subcloning pUC18 and pUC19 plasmids were used. Using a C. thermohydrosulphuricum promoter at least ten different sizes of soluble intracellular thermostable, mostly degenerated polypeptides having both alpha-amylase and pullulanase activities and $M_r$ in the range 165 000 to 100 000 were produced. The temperature optimum of these polypeptides was (80-85° C), some 5° C lower than the temperature optimum of the extracellular enzyme from the native host, but the heat stability was the same as that of the native enzyme. The enzymes can be used alone or in combination with a glucogenic or a maltogenic enzyme for effective hydrolysis of starch and pullulan.

EP 0 402 092 A2

# ALPHA-AMYLASE-PULLULANASE ENZYME

The invention relates to an alpha-amylase-pullulanase enzyme produced by recombinant DNA techniques. A DNA sequence coding for the enzyme having alpha-amylase and pullulanase activity as well as a recombinant DNA molecule comprising the same sequence and a clone of a recombinant bacterium expressing the enzyme are disclosed. Methods for selecting the DNA-sequence for the preparation of the recombinant DNA molecule and the recombinant clone as well as methods for the production and the use of the enzyme are provided in the present invention.

Starch is composed of glucose units linked together to form chains of varying length through alpha-1,4-linkages. Most of the chains are branched through alpha-1,6-linkages, the number of which amounts to about 3-4% of the total linkages. Pullulan is also composed of glucose, but in pullulan every third linkage between the constituent glucose units is an alpha-1,6-linkage the rest of the linkages being of the alpha-1,4-type. Alpha-amylases [EC 3.2.1.1] attack the alpha-1,4-linkages in starch, whereas pullulanases [EC 3.2.1.41] attack the alpha-1,6-linkages in pullulan and starch. Alpha-amylases are needed for the liquefaction and saccharification of starch, whereas pullulanases are used for its debranching.

It is advantageous for the starch hydrolysis process if the enzymes used are active and stable at high temperatures. The alpha-amylases of Bacillus amyloliquefaciens and B.licheniformis have temperature optima at about 70 °C and 92 °C, respectively. The temperature optima of pullulanases are usually lower, e.g. those of Klebsiella pneumoniae (Aerobacter aerogenes) and B.acidopullulyticus have optima at about 55 °C and 60 °C, respectively. If both an alpha-amylase and a pullulanase is used in a process it may be necessary to compromise with the optimum activity requirements of the enzymes, or alternatively to use the enzymes in succession and adjust the conditions of the process in between. Neither of these alternatives are advantageous economically or as a process technique.

It would be more convenient and more economic if only one thermostable enzyme having both of these activities could be used. Enzymes having both alpha-amylase and pullulanase activities have been described recently. Takasaka (1985) has disclosed a pullulanase-like enzyme with alpha-amylase activity derived from Bacillus subtilis (US 4,657,865). Plant et al. have disclosed the properties and methods for partial purification of an extracellular Thermoanaerobium pullulanase having also alpha-amylase activity (Appl. Microbiol. Biotechnol. (1987) 26, 427-433; Biochem. J. (1987), 246, 537-541).

Melasniemi & Korhola (1986) have disclosed an alpha-amylase-pullulanase enzyme derived from Clostridium thermohydrosulfuricum in the European Patent Application EP 258 050. However, the bacterium is an obligate anaerobic thermophile. Thus, its cultivation requires special anaerobic conditions, which are not normally used in enzyme production. In addition, the bacterium has complex nutritional requirements, which makes the production of the enzyme disadvantageous economically.

By using recombinant DNA techniques proteins which are of interest but obtained with difficulty, can be produced in alternative host organisms, which have less complex nutritional requirements and which are easier to cultivate.

Coleman et al. (1987) have cloned a debranching enzyme from Thermoanaerobium brockii into Escherichia coli and B. subtilis (USP 4,612,287) and Kuriki et al. have transferred a pullulanase gene from Bacillus stearothermophilus into a B. subtilis host (Journal of Bacteriology 170, 1554-1559 (1988)).

The invention seeks to provide, by recombinant DNA techniques, a thermostable alpha-amylase-pullulanase enzyme, that may be capable of liquefying, debranching and saccharifying starch.

The invention also seeks to provide a recombinant DNA molecule that may be capable of being transferred into a host, which may be easy to cultivate, may have no complex nutritional requirements and may produce the desired enzyme after receiving the recombinant DNA molecule.

Thus according to a first aspect of the present invention there is provided a nucleic acid having a sequence which is:

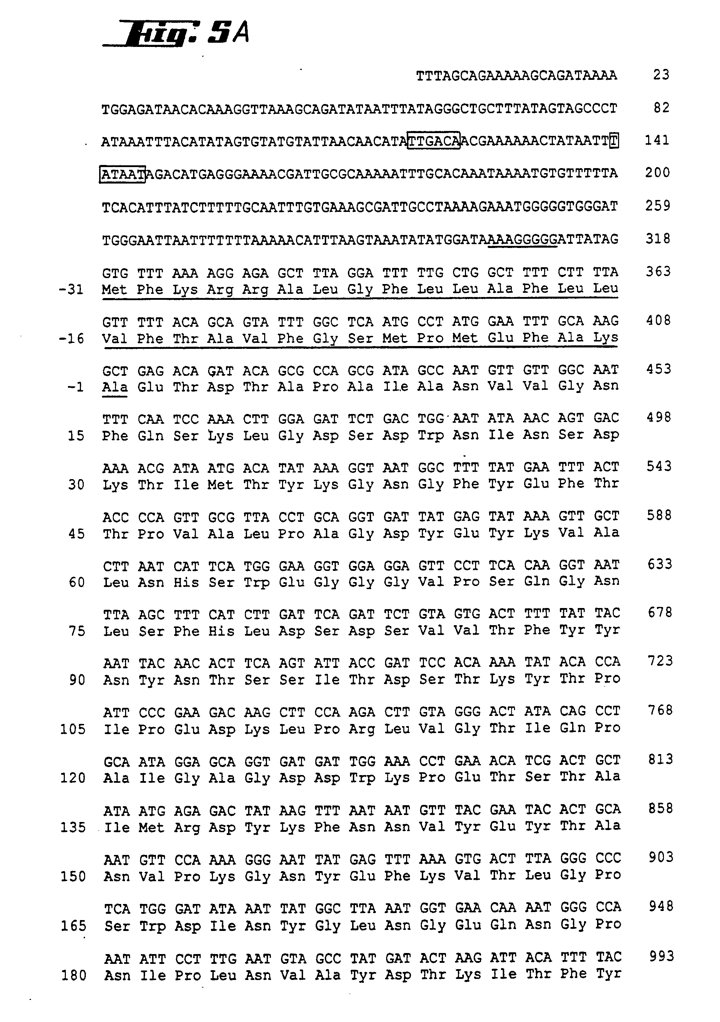

(a) the sequence of Figures 5A to 5F;

(b) a sequence encoding a protein having the amino acid sequence of Figures 5A to 5F;

(c) a sequence as in (a) and/or (b) with a single or multiple base substitution, deletion, insertion or inversion; or

(d) a portion of a sequence in (a), (b) or (c).

The nucleic acid is preferably DNA such as a DNA fragment or sequence. The invention thus encompasses an isolated and sequenced DNA fragment coding for a thermostable enzyme with pullulanase/alpha-amylase activity. The nucleic acid (e.g. DNA fragment) may be in purified form. The DNA fragment is preferably capable of being combined with a suitable vector and introduced into a suitable host organism by the vector or integrated into the chromosome of the host, to produce an enzyme (or a preparation comprising an enzyme) having both pullulanase and alpha-amylase activities, the nucleic acid (e.g. DNA fragment) comprising any of the following DNA sequences:

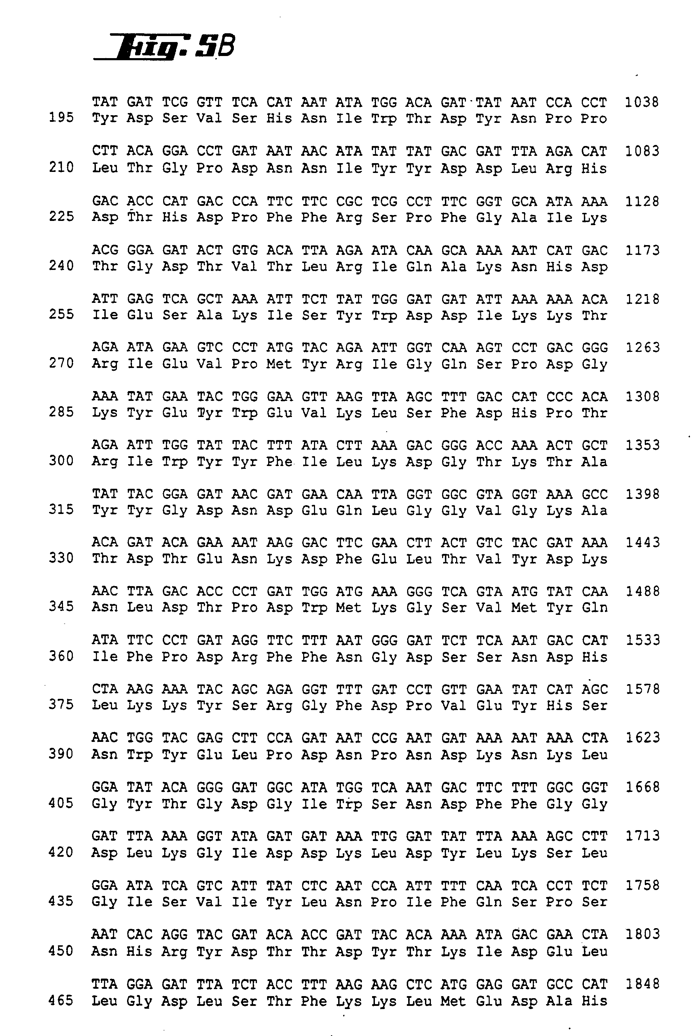

(a) the DNA sequence of Figure 5;

(b) a DNA sequence coding for an enzyme having the amino acid sequence of Figure 5;

(c) variants of the foregoing DNA sequences having single or multiple base substitutions, deletions, insertions or inversions which code for a polypeptide having both pullulanase and alpha-amylase activities; and

(d) portions of the foregoing DNA sequences which code for polypeptides (having such activities).

Where fragments or portions of sequences (whether nucleic acid or amino acid) are referred to in the specification these are preferably 30, 150 or 300 or more base pairs long or 10, 50 or 100 or more residues long, respectively.

The invention also provides a recombinant DNA molecule comprising the DNA fragment as well as a recombinant clone, i.e. a host expressing the enzyme.

The invention also relates to an enzyme preparation comprising the enzyme which has characteristics distinct from previously known enzymes with pullulanase/alpha-amylase activity.

Methods for selecting the DNA sequence, cloning methods as well as methods for expressing and producing the desired enzyme are also provided.

The use of the enzyme (or preparation) having this activity for effective hydrolysis of starch is also disclosed.

The present invention thus provides novel nucleic acid (e.g. a DNA fragment) encoding for an enzyme having both pullulanase and alpha-amylase activities. This DNA fragment may comprise the nucleotide sequence shown in Figure 5, or variants of said nucleotide sequence (single or multiple) having base substitutions, deletions, insertions or inversions, which may be derived from natural sources or created (e.g. synthetically) by laboratory methods known to those skilled in the art. The variants usually code for polypeptides which have the above discussed two enzymatic activities. Portions or fragments of the (nucleic acid) sequence encoding polypeptides having the two enzymatic activities are also within the scope of this invention, as are proteins encoded thereby.

It will be understood that the protein or enzyme encoded by the nucleic acid (or recombinant DNA molecule) of the first aspect may be taken to exclude the protein or enzyme naturally occurring in the cell or micro-organism.

The nucleic acid (e.g. DNA fragment) in question can be used to express a protein (enzyme) having these activities when introduced into a suitable organism in a e.g. (DNA) vector or when integrated into the chromosome of the host.

The DNA fragment (which may be inserted into a vector to obtain a recombinant DNA molecule) suitably comprises an EcoRI fragment of approximately 7 kb (or portions thereof) derived or obtainable from a microorganism of the genus Clostridium and wholly encodes for an enzyme with pullulanase and alpha-amylase activities.

More specifically the nucleic acid (e.g. DNA fragment), comprises a DNA sequence (or portions thereof) coding for an enzyme with pullulanase and alpha-amylase activities, said fragment being derived from a Clostridium strain, preferably Clostridium thermohydrosulfuricum (e.g. DSM 3783).

The DNA fragment in question preferably comprises a promoter sequence from Clostridium, which can be used for the transcription of the DNA fragment.

The invention also embraces a recombinant DNA molecule comprising at least one DNA fragment coding for an enzyme having both pullulanase and alpha-amylase activities comprising the nucleic acid (e.g. DNA) sequence specified above.

A second aspect of the present invention relates to a vector comprising the nucleic acid of the first aspect. The preferred vectors of the present invention include lambda phages, such as lambda gt11, or pUC-plasmids, such as pUC18 and pUC19. The vectors are generally used to isolate the nucleic acid, such as DNA fragment, and to transfer it into the host cell.

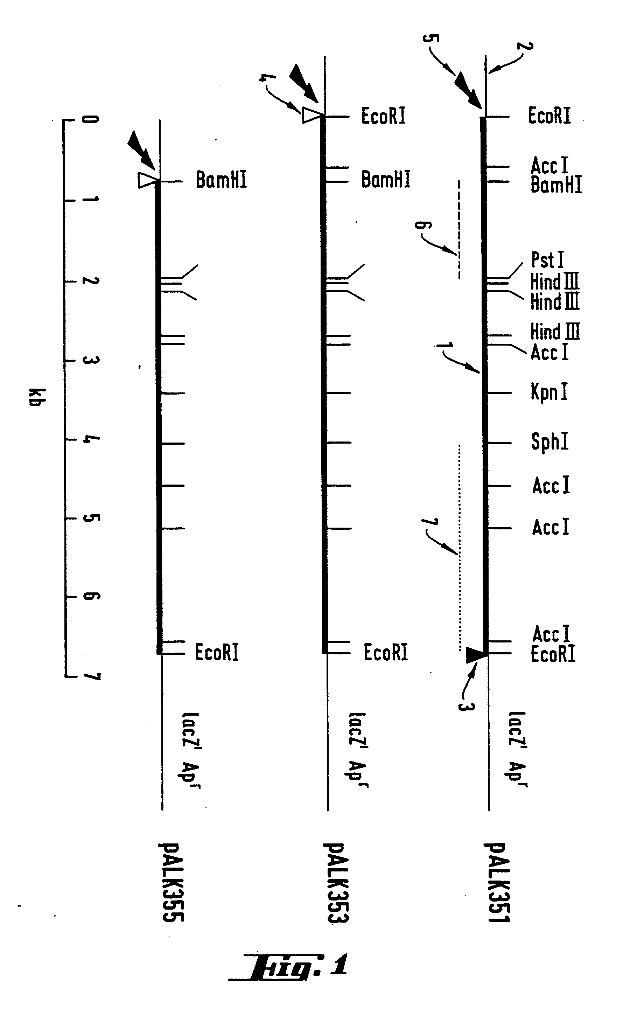

The recombinant DNA molecules that have been constructed in accordance with the present invention are pALK351, pALK352, pALK353, pALK354 and pALK355. pALK351 comprises an approximately 7 kb EcoRI fragment from C. thermohydrosulfuricicum inserted into the EcoRI site of pUC18 in the orientation given in Figure 1. pALK352 is as pALK351, but the orientation of the insert is the opposite. pALK353 comprises the same EcoRI insert in the EcoRI site of pUC19 in the orientation given in Figure 1. pALK354 is as pALK353, but the orientation of the insert is the opposite. pALK355 was derived from pALK353 by deleting its EcoRIBamHI fragment of about 0.8 kb.

The nucleic acid, e.g. a recombinant DNA molecule, of the present invention suitably comprises the promoter sequence from a Clostridium strain, such as Clostridium thermohydrosulfuricum, preferably Clostridium thermohydrosulfuricum DSM 3783 and/or the E. coli beta-galactosidase promoter, but other suitable promoters known in the art may be used.

The recombinant DNA molecule preferably comprises a DNA sequence, or a portion thereof encoding for an enzyme with pullulanase and alpha-amylase activities, which is derivable from Clostridium thermohydrosulfuricum DSM 3783. Suitably the recombinant DNA molecule comprises a promoter sequence from a Clostridium strain, such as the promoter sequence of Clostridium thermohydrosulfuricum DSM 3783. The recombinant DNA molecule preferably comprises pALK353 deposited as a recombinant clone in E. coli (DSM 3533).

The recombinant DNA molecule may comprise as an insert at least one DNA fragment coding for an enzyme with alpha-amylase and pullulanase activities joined with a suitable vector and introduced into a suitable host organism by the vector or integrated into the chromosome of the host, the recombinant DNA molecule comprising a DNA sequence which is:

(a) the DNA sequence of the first aspect;

(b) the DNA sequence of the insert of pALK353 (DSM 5355); or

(c) a DNA sequence which hybridises to a sequence in (a) or (b) and which encodes for an enzyme with alpha-amylase/pullulanase activities.

The (recombinant) host is suitably a bacterium (such as E. coli or Bacillus), yeast or fungus. A preferred (recombinant) host is E. coli, such as the strains Y1090 (r-), Y1089 (r-) or JM109.

Thus the (recombinant) clone so formed preferably comprises at least one DNA fragment derived from a micro-organism of the genus Clostridium, or a portion thereof, encoding for an enzyme with pullulanase and alpha-amylase activities, which is suitably derivable from Clostridium thermohydrosulfuricum (e.g. DSM 3783).

The clone preferably comprises a promoter sequence from a Clostridium strain, such as Clostridium thermohydrosulfuricum (e.g. DSM 3783).

Recombinant clones that may be prepared in accordance with the present invention suitably comprise at least one DNA fragment coding for the enzyme described above. These fragments can be integrated with the chromosomal DNA of the host or combined with a suitable vector.

The recombinant DNA molecule pPALK353 comprising the DNA fragment coding for an enzyme with alpha-amylase/pullulanase activities has been deposited at the Deutsche Sammlung fur Microorganismen (DSM), as a recombinant clone of E.coli JM109, which has received the number DSM 5355.

The recombinant DNA molecules of the present invention may comprise DNA fragments coding for an enzyme with alpha-amylase/pullulanase activities which have been joined together with suitable DNA vectors or the genomes of other organisms to give them the capacity of being transferred and/or maintained in a host and its progeny. Such recombinant DNA molecules comprising a DNA sequence may be selected from:

(a) the DNA sequence of Figures 5A to 5F;

(b) the DNA sequence of the alpha-amylase-pullulanase gene contained on the insert in pALK353 (DSM-5355);

(c) a DNA sequence coding for enzymes having the amino acid sequence of Figures 5A to 5F; and

(d) DNA sequences which hybridise to any of the foregoing DNA sequences and which code for an enzyme with alpha-amylase/pullulanase activities.

The recombinant DNA molecules of the present invention can be transferred into, or their inserts integrated into, the genome of several possible hosts.

Thus a third aspect of the present invention relates to a host transfected with a vector of the second aspect. The hosts can be bacteria, yeasts, fungi or animal cell lines. The preferred bacterial hosts are E. coli or Bacillus strains. Suitable Bacillus strains are B. subtilis, B. brevis, B. amyloliquefaciens, B. licheniformis and B. megaterium. The E. coli strains used to reduce this invention into practice are Y1090 (r-) and Y1089 (r-), which are used for cloning and the strain JM109, which is used as the host for expression and production.

The present invention also provides a new enzyme preparation or enzyme produced by recombinant DNA techniques having both alpha-amylase and pullulanase activities (Figure 2) and comprising polypeptides having $M_r$ from 165 000 down to 100 000 as seen from Figure 2 and Figure 3. These Figures show that the enzyme preparation or enzyme having both alpha-amylase and pullulanase activity may be composed of polypeptides much shorter than full length polypeptides having an $M_r$ or 165 600 as deduced from the DNA sequence. Therefore, enzyme preparations enzymes containing portions of the above defined amino acid sequence and exhibiting the above defined activities are within the scope of this invention. The enzyme preparation or enzyme of this invention may have a temperature optimum of 80-85 °C (Figure 4) and a heat stability as shown in Table 1.

The enzyme in question can be encoded from a DNA fragment having the DNA sequence defined above and it may have an amino acid sequence as defined above. The enzyme is usually associated with the cells, when produced by E. coli. The enzyme thus produced may lack the carbohydrate characteristic of the enzyme produced by the native host, but in spite of this the enzyme and the preparation comprising it is active. Although the protein produced by recombinant DNA techniques can be degenerated, its activity and properties are not significantly altered.

The present invention also provides methods for isolating a DNA sequence (fragment) coding for the enzyme with pullulanase and alpha-amylase activity. A preferred method comprises:

(a) difesting chromosomal DNA from a Clostridium strain, preferably Clostridium thermohydrosulfuricum (DSM 3783);

(b) inserting resultant DNA fragments into a suitable vector to obtain a recombinant DNA molecule;

(c) infecting a suitable host with the recombinant DNA molecule;

(d) screening for the presence of the inserted DNA fragment by a method which is preferably an immunological, enzymatical and/or hybridisation method.

In the immunological screening method a novel rabbit alpha-amylase-pullulanase antiserum, prepared against the enzyme of the native host can be used. The invention thus encompasses an antibody specific for the protein encoded by nucleic acid (e.g. DNA) of the first aspect, such as specific for the enzyme (having alpha-amylase and pullulanase activity) of the present invention.

In the enzymatic screening method the enzyme activity can be detected on plates overlayed with a starch containing layer and starch digestion may be indicated with iodine.

In the hybridisation method the alpha-amylase-pullulanase gene can be detected on plates overlayed with a starch containing layer and starch digestion may be indicated with iodine.

In the hybridisation method the alpha-amylase-pullulanase gene can be detected by hybridising with probe(s) constructed synthetically by converting a part of the amino acid sequence of the enzyme protein into corresponding DNA sequences. The probes can then be used to identify clones containing homologous DNA sequences from the DNA library prepared from a Clostridium strain, preferably Clostridium thermohydrosulfuricum DSM 3783.

The DNA fragment with the desired properties can be used to construct several different recombinant DNA molecules using techniques known to those skilled in the art.

The enzyme preparation or enzyme of the present invention with pullulanase and alpha-amylase activity can be produced by several methods, one of which comprises:

(a) selecting a DNA fragment encoding for the enzyme;

(b) inserting the fragment into a suitable vector to produce a recombinant DNA molecule;

(c) introducing the recombinant DNA molecule into a suitable host to obtain a recombinant clone;

(d) cultivating the recombinant clone in a suitable medium; and

(e) Recovering the enzyme after cultivation.

When the host is E. coli, the enzyme preparation or enzyme with pullulanase and alpha-amylase activity can be recovered after being released from the cells. A host capable excreting the enzyme, e.g. a Bacillus strain, can also be used.

The invention also encompasses an enzyme preparation (e.g. produced by recombinant DNA techniques) comprising an enzyme having both alpha-amylase and pullulanase activities, an $M_r$ of approximately 165 000 - 100 000, a temperature optimum of 80-85 °C and a heat stability according to Table 1. This is preferably a soluble intracellular enzyme produced by Escheridiia coli.

The invention will be described by way of example with reference to the accompanying drawings in which:

Figure 1 depicts plasmids pALK351, pALK353 and pALK355 of the present invention together with a restriction map of the subcloned DNA fragment from C. thermohydrosulfuricum;

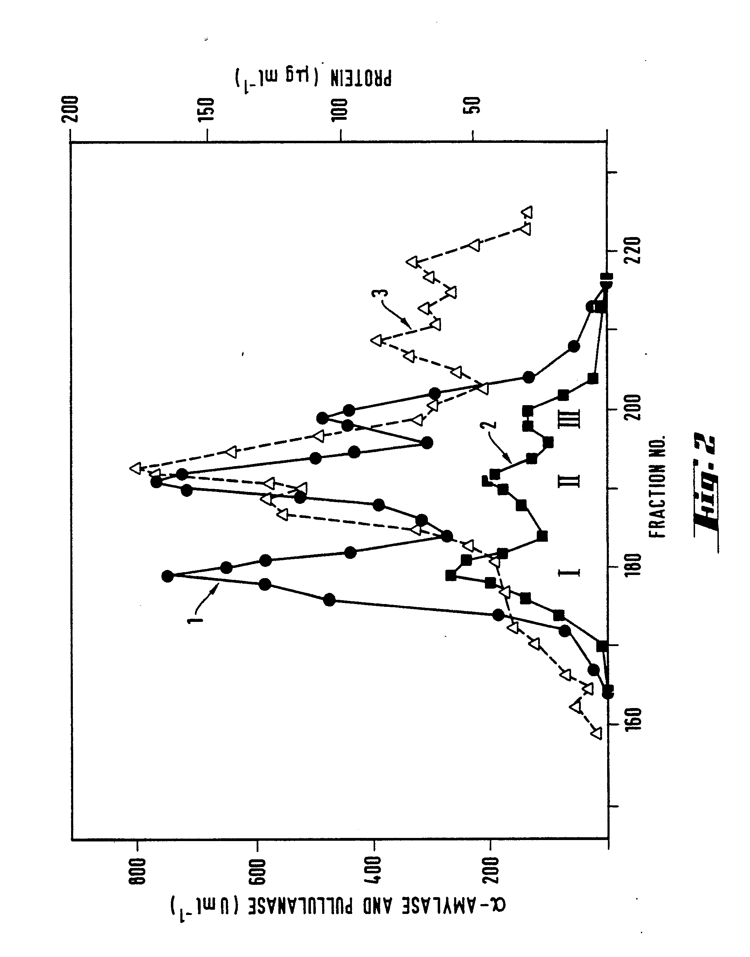

Figure 2 is a graph showing gel filtration of an alpha-amylase-pullulanase produced according to the present invention;

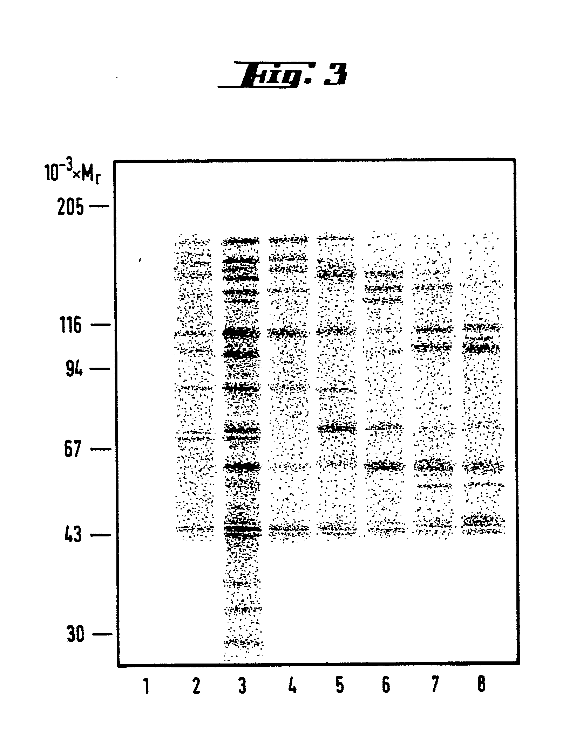

Figure 3 is an immunoblot of an alpha-amylase- pullulanase according to the present invention;

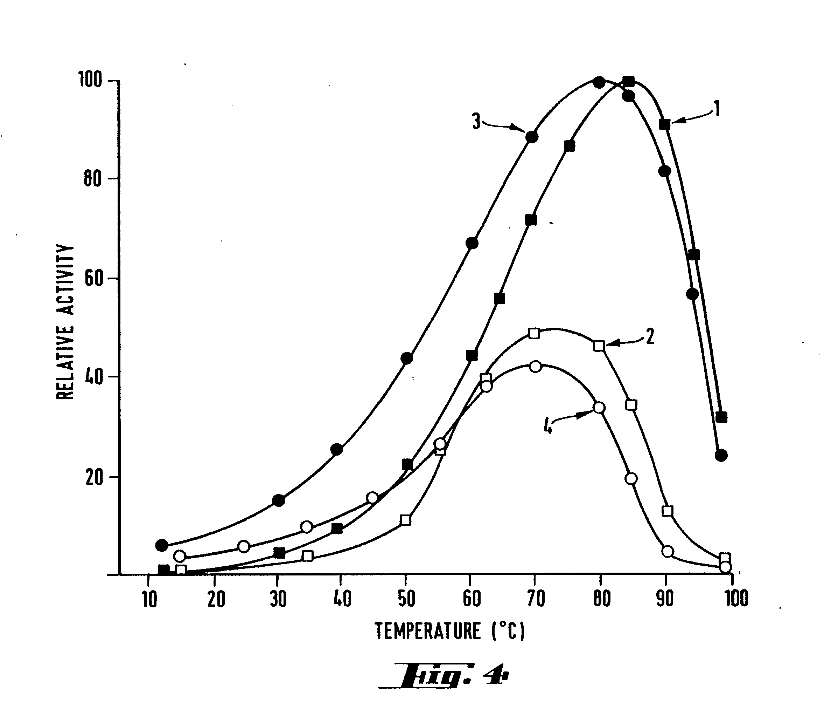

Figure 4 is a graph of temperature against activity of an alpha-amylase-pullulanase produced according to the present invention;

Figures 5A to 5F show the nucleotide sequence of the C. thermohydrosulfuricum alpha-amylase pullulanase gene, together with a deduced amino acid sequence of the enzyme, in accordance with the present invention; and

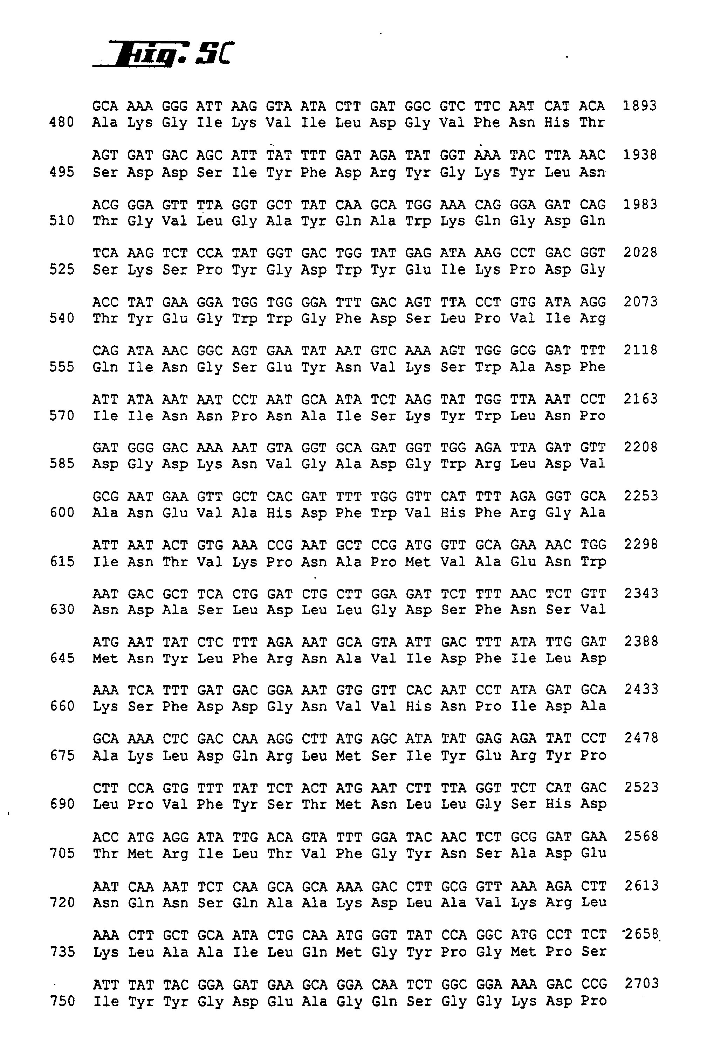

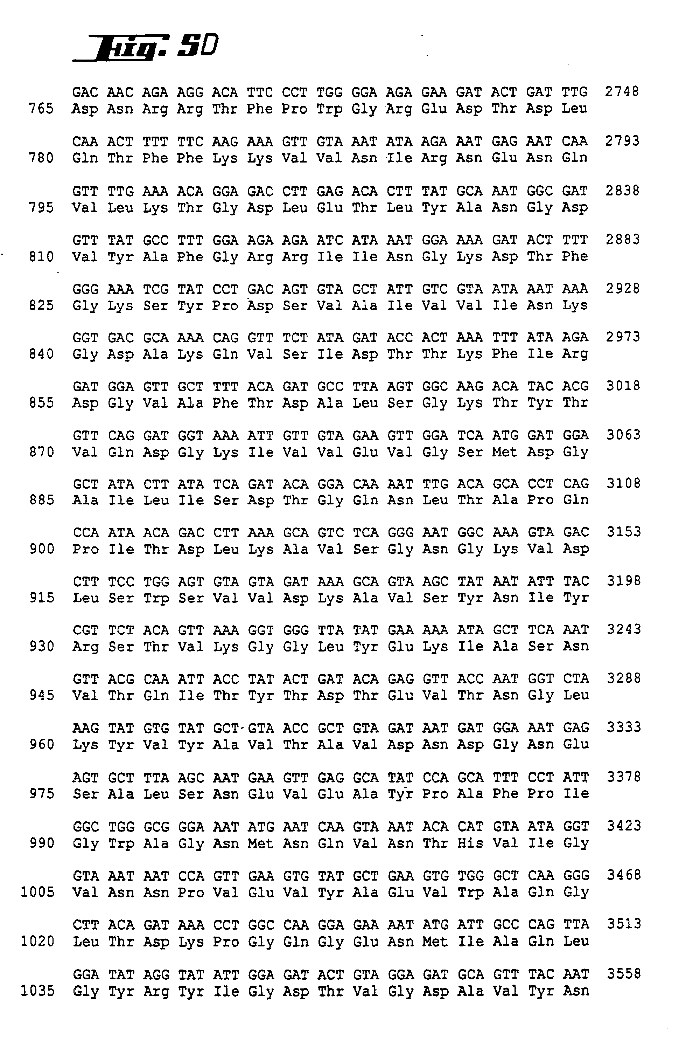

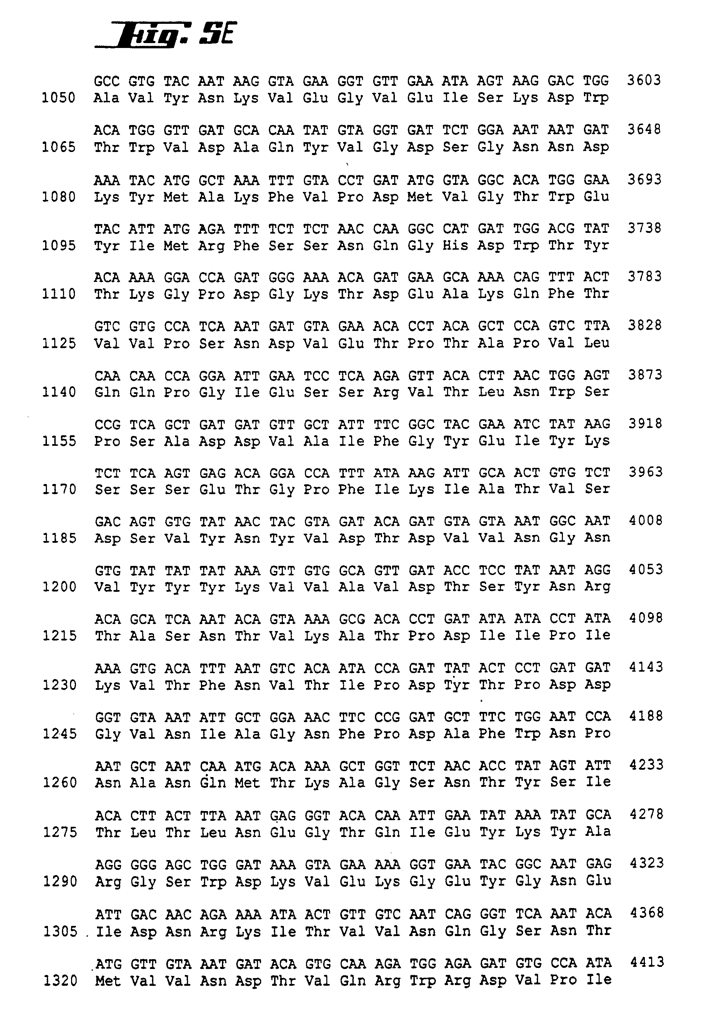

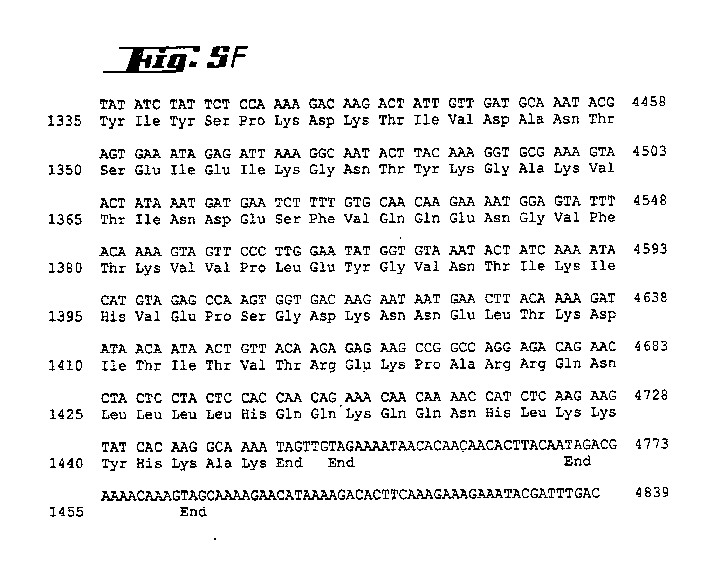

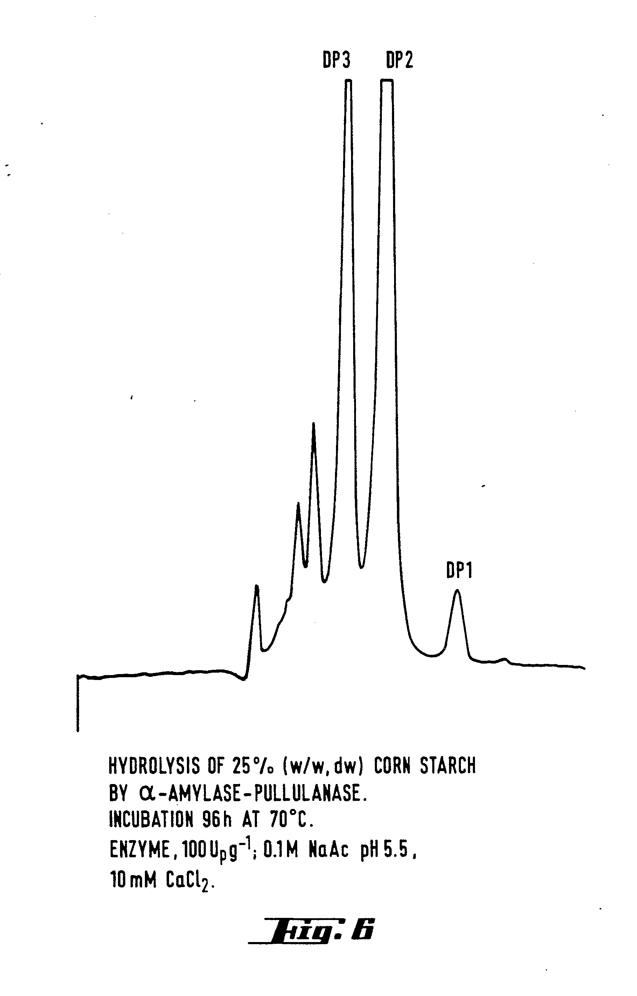

Figure 6 shows the hydrolysis of corn starch, by an alpha-amylase-pullulanase (alone) according to the present invention, to a mixture containing about 80% maltose/maltotriose.

In a fourth aspect the present invention relates to a process for hydrolysing starch, amylase or pullulan, the process comprising contacting starch, amylase and/or pullulan with a protein or an enzyme of the first aspect, preparable by a process as previously described, alone or with a glucogenic or maltogenic enzyme.

A fifth aspect of the present invention relates to a recombinant protein having alpha-amylase and pullulanase activity. The protein is preferably obtainable from a micro-organism of the genus Clostridium, such as Clostridium thermohydrosulfuricum.

A sixth aspect of the present invention relates to a protein, preferably recombinant, such as an enzyme having both alpha-amylase and pullulanase activity, encoded by a nucleic acid sequence (or a recombinant DNA molecule) as described in the first aspect.

Preferred features and characteristics of one aspect of the present invention are as for another aspect mutatis mutandis.

The invention will now be described by way of example with reference to the accompanying Example, which is provided for means of illustration and is not to be construed as being limiting on the present invention.

## EXAMPLE 1

Production of a thermostable alpha-amylase-pullulanase enzyme; preparation of enzyme by recombinant DNA techniques

### 1. Materials

Bacterial strains, phages, plasmids and culture media. C. thermohydrosulfuricum DSM 3783 (E 101-69) were grown as described in Melasniemi, Journal of General Microbiology 133, 883-90 (1987) for 20 h in 2 1 of medium containing 2% (w/v) glucose. EcoRI digested dephosphorylated lambda gt11 vector DNA and E. coli host strains Y1090 (r- and Y1089 (r-) are contained in the Protoclone lambda gt11 system (Promega). The host strains are restriction minus derivatives of the strains Y1090 and Y1089, the use of which with lambda gt11 has been described in detail by Huynh et al., (DNA Cloning: a Practical Approach, vol. I, pp. 49-78 (1985). Edited by D.M. Glover. Oxford: IRL Press.).

Plasmids pUC18 and pUC19, differing from each other only in the orientation of their polylinkers, were used for sub-cloning in E. coli JM109 (Yanisch-Perron et al., Gene 33, 103-119 (1985)). The polylinkers of the plasmids as well as the unique EcoRI site of lambda gt11 are located within the beta-galactosidase gene adjacent to its promoter enabling recombinant clones having inserts in these cloning sites to be recognised by the X-gal test.

The growth medium used for cultivating E. coli JM109 was Luria-Bertani (Maniatis et al., Molecular Cloning: a Laboratory Manual. Cold Spring Habor, NY: Cold Spring Harbor Laboratory (1982)) containing ampicillin 50-100 g ml$^{-1}$. The medium was used as agar plates or as liquid.

### 2. Methods

#### a) Isolation of DNA

Chromosomal DNA was isolated from C. thermohydrosulfuricum by the phenol method of Amundsen & Neville (Microbios 24, 29-39 (1979)) modified as follows. Cells (5 g, wet wt) were incubated for 15 min at 37 °C in 25 ml of 50 mM Tris/HCl pH 8.0 containing 10 % (w/v) sucrose, 50 mM EDTA and lysozyme (5 mg/ml). Sarcosyl was added to 2 % (w/v) and the incubation was continued for 30 min at 65 °C. The isolated DNA was treated with proteinase K and extracted with phenol and chloroform. The solution of the DNA in TE (10 mM Tris/HCl pH 8.0, 0.1 mM EDTA) was adjusted to a refractive index of 1.3804 with CsCl and centrifuged at 40 000 rpm for 20 h at 20 °C in a Kontron TFT 50.13 rotor with full tubes. The DNA was recovered from the bottom fractions and dialyzed against TE. Lambda DNA was extracted from DNase and RNase treated polyethylene glycol precipitated phage particles (Maniatis et al., 1982). Plasmid DNA was isolated by the method of Birnboim & Doly, Nucleic Acids Research 7, 1513-1523 (1979)). For rapid screening of clones the method of Holmes & Quigley (Analytical Biochemistry 114, 193-197 (1981)) was used.

b) DNA manipulations

A buffer containing 6 mM Tris/HCl pH 7.9, 50 mM NaCl, 6 mM $MgCl_2$ and 6 mM β-mercaptoethanol was used for all restriction enzymes except EcoRI, for which 1/10 vol. 1 M Tris/HCl pH 7.6 was added to the buffer. Analysis of restriction fragments and restriction mapping was done as described by Maniatis et al. (1982). For transformation of E. coli the method of Hanahan (DNA Cloning: a Practical Approach, vol. I, pp. 109-135 (1985). Edited by D.M. Glover. Oxford: IRL Press) was used. Restriction enzymes, T4 DNA ligase and DNA standards were from Boehringer Mannheim.

c) Cloning and sub-cloning

EcoRI digested chromosomal DNA was fractionated by sucrose density gradient centrifugation (maniatis et al., 1982). Fragments between 2-9 kb were pooled and ligated to the EcoRI site of lambda gt11 DNA. The DNA was packed in vitro into particles by using packagene (Promega) packaging extracts. Positive plaques on a lawn of Y1090(r-) were screened by using a rabbit α-amylase-pullulanase antiserum essentially as described by Huynh et al. (1985). The immunocomplexes were visualized by using goat anti-rabbit IgG horse raddish peroxidase conjugate (Bio-Rad) together with the chromogenic substrate 3-amino-9-ethylcarbazole (Sarvas & Nurminen, Enterobacterial Surface Antigens: Methods for Molecular Characterisation, pp. 123-137 (1985). Edited by T.K. Korhonen, E.A. Dawes & P.H. Mäkelä. Amsterdam: Elsevier). Screening for enzyme activity was done by over laying the plates with 5 ml of 0.8 % melted agar in 0.2 M sodium acetate pH 5.2 containing 0.05 % soluble starch (Merck 1252) and 2 mM $CaCl_2$, incubating for 16 h at 55 °C and staining with iodine vapour. Several positive clones were amplified and concentrated phage stocks were prepared by polyethylene glycol precipitation (Maniatis et al., 1982). recombinant DNA extracted from aliquots of the stocks was digested with EcoRI and the sizes of the inserts were analyzed. Y1089(r-) was lysogenized with recombinant phage (Huynh et al., 1985) and phage DNA was prepared from induced cells. The insert was excised by EcoRI, separated from the lambda arms by preparative agarose gel electrophoresis and sub-cloned in the EcoRI site on the polylinker of the pUC plasmids.

d) Oligonucleotide hybridization

Knowing the $NH_2$-terminal amino acid sequences of the extracellular enzyme from the native host (Melasniemi, 1988, Biochemical Journal 250, 813-818) and its satellite protein (Melasniemi, unpublished) 27-mer oligonucleotide mixtures complementary to all possible codon combinations for each of the $NH_2$-terminal sequences were synthesized using a 381A DNA Synthesizer (Applied Biosystems). In the case of the enzyme probe the mixture contained one perfectly matching oligonucleotide species out of a total of 36 864 species, in the case of the satellite protein probe one out of 512. The oligonucleotides were labeled with [$\gamma$-$^{32}$P]ATP using polynucleotide kinase (Maniatis et al., 1982). Hybridization was done at 60 °C directly in a dried 0.8 % agarose gel (Miyada & Wallace, Methods in Enzymology 154, 94-107 (1987)) with restriction fragments of the sub-cloned insert resolved on the gel. To eliminate unspecific binding the gel was washed with 0.9 M NaCl in 90 mM sodium citrate pH 7.0 for a total of 2 h at room temperature, five minutes at 68 °C and finally for 15 min at 45 °C.

e) DNA sequencing

The nucleotide sequence of the α-amylase-pullulanase gene was determined by the M13-dideoxy method (Sanger et al., Proc. Nat, Acad. Sci. USA 74, 5463-5467 (1977)). The whole sequence was determined in both directions with overlapping partial sequences.

f) Preparation of cell-free extracts and localization of the enzyme

Transformed JMIO9 cultures (200 ml) were centrifuged (10 000 g, 10 min), cells were resuspended with 20 mM sodium acetate pH 5.6 to 25 ml and passed four times through a French pressure cell (Aminco) at 4 °C using a pressure of about 100 MPa. Soluble and particulate fractions were separated by ultracentrifugation (165 000 g, 90 min, at 4 °C) and the particulate pellet was resuspended in the same volume of the buffer. Outer and inner membrane fractions were obtained from the crushed cells as described by Smit et al. (Journal of Bacteriology 124, 942-958 (1975)), except that a protease inhibitor cocktail of phenylmethylsulphonyl fluoride, benzamidine/HCl and N-p-tosyl-L-lysine chloromethyl ketone (each 1 mM) and pepstatin A (10 M) was included in all solutions, and possible unbroken cells were separated by centrifuging for 20 min at 1 200 g. Periplasmic proteins were released by osmotic shock (Neu & Heppel, Journal of Biological Chemistry 240, 3685-3692 (1965)) and by a lysozyme-EDTA treatment, in which centrifuged cells were resuspended in 10 mM Tris/HCl pH 8.0 containing 1 mM EDTA, 0.5 M sucrose and lysozyme (5 mg/ml), incubated for 15 min at 37 °C and diluted with an equal volume of water, after which the spheroplasts were separated by centrifugation (10 000 g, 6 min). The periplasmic and intracellular marker enzymes, alkaline phosphatase and glucose-6-phosphate dehydrogenase, respectively, were assayed as described by Malamy & Horecker (Biochemistry 3, 1889-1893 (1964)).

g) Binding of $^{14}C$-starch

Samples boiled for 2.5 min in the sample buffer were resolved on a 10 % SDS-PAGE gel (Laemmli, Nature, London 227, 680-685 (1970)) and blotted on nitrocellulose essentially as described by Sarvas & Nurminen (1985). The filters were gently shaken, first for 2 h at room temperature in 50 mM Tris/HCl pH 8.0 containing 150 mM NaCl and 5 % (w/v, dry wt) skim milk for quenching, and then for 16 h on ice with 35 µCi of [$^{14}C$(U)]-starch (New England Nuclear) in 10 ml of 100 mM sodium acetate pH 5.6 containing 2 mM $caCl_2$, 0.1 mM EDTA and 50 mM NaCl. The filters were washed with ice cold water, dried and exposed to a Kodak X-Omat XR film.

h) Enzyme and protein assays

α-Amylase and pullulanase activities were assayed by measuring the amount of reducing sugar released in 15 min from amylose or pullulan as earlier described (Melasniemi, 1987a), except that a temperature of 80 °C and a $CaCl_2$ concentration of 10 mM was used. One unit of activity is defined as the amount of enzyme releasing 1 nmol of reducing sugar min per minute in the assay. Protein was measured by the Bio-Rad assay using ovalbumin as a standard.

i) Hydrolysis of starch

Pelleted cells of E. coli JM109 carrying pALK355 were resuspended (about 1 g of wet cells to 1 ml) in pH 5.0 and pH 5.5 buffers of 100 mM sodium acetate, 10 mM $CaCl_2$, and cell-free extracts were prepared. The soluble fraction of the extracts were incubated for 10 min. at 70 °C to denature and precipitate heat labile E. coli proteins. The precipitate was removed by centrifugation and washed once with the buffer. The respective supernatants and washings were combined to get two enzyme preparations. The activity of the pH 5.0 preparation was about 80 kU/ml, as expressed in pullulanase units, and the activity of the pH 5.5 preparation about 100 kU/ml.

The pH 5.0 enzyme preparation was used to study the effect of the α-amylase-pullulanase on glucose formation from 25 % (w/w, dw) Zulkowsky starch (Merck) by Aspergillus niger glucoamylase (Table 2). The pH 5.5 enzyme preparation was used to study the effect of the α-amylase-pullulanase on maltose formation from the same substrate by Bacillus stearothermophilus maltogenic α-amylase (Table 3). This preparation was also used alone to hydrolyse 25 % (w/w, dw) corn starch (Sigma), gelatinized by heating in a microwave oven in the pH 5.5 sodium acetate buffer. The percentages of the products formed were calculated by comparing the amounts of the products formed to the amount of glucose released from the enzymatic hydrolysates by a following mild acid hydrolysis 0.5 M HCl, 3 h, 100 °C). The amounts of glucose and maltose/maltotriose in the Zulkowsky starch hydrolysates were determined enzymically by the Boehringer-Mannheim maltose/glucose UV-method. (Boehringer-Mannheim GmbH, Biochemica, Mannheim. Maltose/glucose UV-method. In food analysis using single reagents, pp. 51-52 (1984)). The corn starch hydrolysate was analyzed by HPLC, using a Macronex IC-8101 (Interaction) $Ca^{2+}$-column eluted by $H_2O$ (0.4 ml/min) at 85 °C.

**Results**

a) Cloning of the amylase gene and its promoter region

The C. thermohydrosulfuricum genomic library in lambda gtll had a titre of 9 x $10^5$ plaque forming units per µg of packaged DNA and it contained about 85 % recombinant phage particles, approximately 1 % of which caused the production of α-amylase-pullulanase. Screening the plates first immunologically and subsequently for enzyme activity revealed roughly equal numbers of positive clones, most of which were detected by both methods. Because the immunological method gave clearer signals and required no replication of the plates, it was used to purify. several positive plaques. All purified clones had the same 7 kb insert, which was subsequently sub-cloned in both orientations into the EcoRI site of pUC18 to give the plasmids pALK35l and pALK352 and into the EcoRI site of pUC19 to give the plasmids pALK353 and pALK354.

Fig. 1. shows the restriction map of the 7 kb C. thermohydrosulfuricum DNA fragment in the plasmids pALK351 and pALK353. Insert DNA is shown as a bold line (1); vector DNA as a thin line (2); polylinker of pALK351 is marked by (3); polylinker of pALK353 is marked by (4); the E. coli β-galactosidase promoter is the arrow (5). The plasmid pALK355 (Fig. 1) was obtained from pALK353 by deleting the EcoRI-BamHI fragment adjacent to the β-galactosidase promoter. The regions hybrizing to the α-amylase pullulanase and the satellite protein probes are indicated by a broken line (6) and a dotted line (7) respectively. The restriction sites shown are also found in the oppositely oriented pclylinkers of the pUC plasmids.

Both lambda gt11 and the PUC vectors used are able to express genes inserted in the right reading frame as hybrid proteins from the β-galactosidase promoter upstream of the cloning sites. However, the α-amylase-pullulanase gene is transcribed from a C. thermohydrosulfuricum promoter on the 7 kb insert, because the amount of enzyme activity produced by the recombinant PUC plasmids was not affected by changing the orientation of the insert.

b) Localization of the amylase gene

The extracellular α-amylase-pullulanase of C. thermohydrosulfuricum DSM 3783 occurs in the culture medium in tight, apparently equimolar association with a (Mr 24 000) protein, the α-amylase-pullulanase satellite protein (Melasniemi, 1988). The synthesized 27-mer oligonucleotide probes for the DNA sequences coding for the $NH_2$-terminal ends of the enzyme and the satellite protein hybridized specifically to the 1.2 kb BamHI-PstI fragment and the 2.6 kb SphI-EcoRI fragment of PALK 353, respectively (Fig. 1). Deleting the 0.8 kb EcoRI-BamHI fragment from pALK353 (Fig. 1) by cutting the polylinker and the insert with BamHI and religating to obtain the plasmid pALK355 had no effect on the enzyme activity produced. In contrast, if the 2.6 kb SphI-EcoRI fragment was deleted from pALK351 (Fig. 1) by cutting with SphI and religating, no activity was produced. Together with the hybridization results, these results indicate that the promoter region and the 5′-end of the α-amylase-pullulanase structural gene are located on the 1.2 kb BamHI-PstI fragment of pALK353 and that the gene extends onto the 2.6 kb SphI-EcoRI fragment.

c) DNA sequence of the amylase gene

The complete nucleotide sequence of the alpha-amylase-pullulanase gene with a deduced amino acid sequence is shown in Fig. 5. Numbering of the amino acids (+1) starts with the first amino acid of the amino terminal end of the extracellular enzyme from the native organism (Melasniemi, Biochem. J. (1988), 250, 813-818). The consensus promoter sequences for RNA-polymerase recoqnition and binding are boxed and a likely ribosomal binding site followed by a putative signal sequence of the enzyme (amino acids -31 through -1) are underlined. An open reading frame of 4425 nucleotides starting with GTG and terminating with TAG, was present. This corresponds to a polypeptide consisting of 1475 amino acids and having an $M_r$ of 165 600. The amino acid sequence corresponding to $NH_2$-terminal end of the extracellular enzyme from the native host was preceded by a putative signal sequence, a hydrophobic stretch of 31 amino acids.

d) Localization of the enzyme in E. coli

During growth of E. coli JM109 carrying pALK355, pullulanase and α-amylase activities were proportional to the cell mass and reached 2.1 and 0.64 kU $ml^{-1}$, respectively, after 18 h. This is slightly more than is produced by the original host (Melasniemi, 1987a). Only about 2 % of the total enzyme activity was found in the culture medium. No activity was released by washing the cells with 10 mM Tris/HCl pH 8.0 and intact washed cells showed less than 1 % of the total activity. The bulk of the enzyme activity (98 %) was manifested only after complete disintegration of the cells by French pressing.

About 85 % of the activity of the cell-free extracts was found in the soluble fraction and about 15 % sedimented on ultracentrifugation. However, when possible association of the sedimented enzyme with outer or inner membrane vesicles was tested by separating the vesicles by sucrose density gradient ultracentrifugation no amylase activity was found in the vesicular fractions, and instead the activity was found exclusively in the top fractions of the gradients. The possibility of a periplasmic location was tested. Spheroplasting the cells by lysozyme-EDTA released 80 % of the periplasmic marker enzyme, alkaline phosphatase, less than 1 % of the intracellular marker enzyme glucose-6-phosphate dehydrogenase, and less than 3 % of α-amylase-pullulanase. Osmotic shock released more than 75 % of the alkaline phosphatase, slightly less than 3 % of the glucose-6-phosphate dehydrogenase and about 10 % of the amylase.

The α-amylase-pullulanase produced by C. thermohydrosulfuricum (DSM 3783) is bound to the cell surface or is released into the medium depending on the cultural conditions used (Melasniemi, 1987a). The present results are most consistent with the idea that the enzyme is produced in E. coli as a soluble intracellular protein. Some affinity for the cytoplasmic membrane is suggested by both the sedimentation of a part of the enzyme from homogenates and the slightly higher release of the amylase than of cytoplasmic glucose-6-phosphate dehydrogenase from the cells during the spheroplasting and osmotic shock treatments, which may cause some damage to the cytoplasmic membranes.

e) Degeneracy of the amylase produced in E. coli

In Fig. 2. is shown gel filtration of the α-amylase-pullulanase produced in E. coli on combined Superose 12 and 6 columns. A sample (200 µl) from the soluble fraction of a cell-free extract from JM109 carrying pALK355 was run at room temperature through consecutive Superose 12 and 6 columns (Pharmacia) in 100 mM sodium acetate pH 5.6 at a rate of 6 ml/h and 150 µl fractions were collected. In Fig. 2 pullulanase activity is depicted by (1); α-amylase activity by (2) and protein by (3).

Gel filtration resolved the α-amylase-pullulanase activity into three peaks (I-III) each showing both α-amylase and pullulanase activities in an approximate 1/3 ratio.

Approximate $M_r$-values of (I) 330 000, (II) 245 000 and (III) 202 000 were obtained from a linear calibration curve established by using thyroglobulin ($M_r$ 669 000), apoferritin ($M_r$ 443 000), catalase ($M_r$ 232 000 and aldolase ($M_r$ 158 000) as standards. The $M_r$ of the enzyme in peak III may, however, be somewhat over estimated, since deviations from linearity were observed with proteins smaller than aldolase, the smallest protein used for calibration.

Fig. 3 is shown immunoblotting of the α-amylase-pullulanase produced in E. coli. Over ten distinct α-amylase-pullulanase specific bands were resolved from cells carrying pALK355 (Fig. 3). Samples were run on SDS-PAGE (Laemmli, 1970) on a gel containing linear gradients of 4-16 % polyacrylamide and 0-18 % sucrose in the separating gel. Rabbit muscle myosin, E. coli β-galactosidase and Low Molecular Weight Calibration Kit proteins (Pharmacia) were used as standards. Transfer to a nitrocellulose filter and immunostaining was done essentially as described by Sarvas & Nurminen (1985). Lanes: [1-3, cell-free extracts from transformed JM109; 4-8, gel filtration fractions (12.5 µl) from Fig. 2] 1, unfractionated extract/pUC19 (5 µl); 2, particulate fraction/pALK355 (2.5 µl); 3, soluble fraction/pALK355 (2.5 µl); 4, fr179; 5, fr.184; 6, fr.191; 7, fr.196; and 8, fr.199. The authenticity of the bands was confirmed by specific binding of [$^{14}$C]-starch by all of the immunologically detected major bands above $M_r$ 40 000 (results not shown).

The three enzyme peaks obtained by gel filtration (Fig. 2) were composed of different sets of α-amylase-pullulanase polypeptides as judged by the intensities of the bands in the immunoblot in Fig. 3. Peak (I) contained a major band with $M_r$ about 165 000, which was the highest value observed. This value agrees well with the $M_r$ 165 600 calculated for full length polypeptides from the DNA sequence. Peak (II) showed a triplet of bands around $M_r$ 130 000 and peak (III) a doublet around M, 100 000. The last value shows that the polypeptide chain can be broken at more than one third the way along its length without concomitant loss of activity. These $M_r$-values together with those obtained by gel filtration suggest that the enzyme produced in E. coli is dimeric as is the extracellular enzyme produced by the native host (Melasniemi, 1988).

f) Thermal characteristics of the enzyme

The effect of temperature on the activity of the enzyme produced in E. coli is shown in Fig. 4. The soluble fraction of a cell-free extract from JM109 carrying pALK355 was diluted with 20 mM NaAc pH 5.6 to 2.1 kU pullulanase activity, 0.64 kU α-amylase activity and 0.8 mg protein ml/ml and was then assayed for α-amylase (1,2) and pullulanase (3,4) activities as described in the Methods, but using 50 µl samples and various temperatures. The assay was performed in the presence (1,3) or absence (2,4) of 10 mM $CaCl_2$.

For the 15 min reaction times used in the presence of 10 mM $Ca^{2+}$, the optimum of the α-amylase activity was at about 85 °C and the optimum of the pullulanase activity some five degrees lower at about 80 °C. Raising the $Ca^{2+}$ concentration did not shift the optima to higher temperatures, but if $Ca^{2+}$ was omitted the optima were shifted some ten degrees lower and at the same time both activities were reduced to about half of that in the presence of $Ca^{2+}$.

Activation by $Ca^{2+}$ at 80 °C (Fig. 4) was about 290 % for the pullulanase and about 210 % for the α-amylase activity. Over 90 % of the maximal activation was observed with only 0.25 mM $Ca^{2+}$.

Table 1.

Heat stability of the α-amylase-pullulanase produced in E. coli. The soluble fraction of a cell-free extract from JM109 carrying pALK355 was diluted with 100 mM NaAc pH 5.6 to 2.1 kU pullulanase activity, 0.64 kU α-amylase activity and 0.8 mg protein $ml^{-1}$. Replicate samples were incubated at 55-95 °C in the presence or absence of 10 mM $CaCl_2$ for 30 and 120 min. The activity left was assayed as described in the Methods using 50 1 enzyme samples and pullulan as a substrate.

| | Residual activity (%) | | | |
|---|---|---|---|---|
| | 30 min | | 120 min | |
| Temperature (°C) | No $Ca^{2+}$ | 10 mM $Ca^{2+}$ | No $Ca^{2+}$ | 10 mM $Ca^{2+}$ |
| 55 | 93 | 91 | 92 | 90 |
| 60 | 93 | 90 | 94 | 91 |
| 65 | 96 | 89 | 96 | 91 |
| 70 | 94 | 89 | 93 | 91 |
| 75 | 79 | 87 | 65 | 87 |
| 80 | 46 | 84 | 15 | 72 |
| 85 | 8.5 | 75 | 0.9 | 64 |
| 90 | 1.3 | 58 | 0.0 | 22 |
| 95 | 0.5 | 13 | 0.1 | 1.0 |

Thermal stability of the enzyme in the absence of substrate is presented in Table 1. In the absence of $Ca^{2+}$ the enzyme was stable at 65 °C, but was rapidly inactivated at 85 °C. In the presence of 10 mM $Ca^{2+}$, however, more than 60 % of the activity was still left after incubating the enzyme for 2 h at 85 °C.

Considering the marked degeneracy of the enzyme produced in E. coli, its thermal characteristics were suprisingly little affected as compared with the enzyme produced by the native host (Melasniemi, 1987b, Biochemical Journal 246, 193-197): the temperature optimum was only some five degrees lower and the stability the same. Since an intracellular protein in E. coli can fairly safely be assumed to be non-glycosylated, the equal stabilities of the enzyme produced in either host seem to imply that the carbohydrate moiety of the C. thermohydrosulfuricum enzyme has no marked role in the thermostability of the enzyme.

g) Hydrolysis of starch

As seen from Tables 2 and 3, higher amounts of glucose and maltose/maltotriose, were formed from 25 % Zulkowsky starch by A. niger glucoamylase and B. stearothermophilus maltogenic α-amylase, respectively, in the presence of the α-amylase-pullulanase, than in its absence. The soluble Zulkowsky starch was used here as a substitute for liquefied starch. However, it is a non-native starch obtained by a hot glycerol solubilization at 190 °C (Zulkowsky, K.). Verhalten der Stärke gegen Glycerin. Berichte der Deutschen Chemischen Gesellschaft 13, 1395-1398 (1880), and somewhat higher yields can presumably be obtained when ordinary starches are used. Gelatinized 25 % corn starch hydrolysed solemnly by the α-amylase-pullulanase (Fig. 6) yielded a mixture containing about 80 % maltose/maltotriose. The amount of maltose formed in 96 h was 56 % and the amount of maltotriose 26 %. Thus, this single enzyme can be used to effectively convert starch to these sugars.

Table 2.

| The effect of α-amylase-pullulanase (αAP)on glucose formation by glucoamylase. | | |
|---|---|---|
| Substrate, 25 % (w/w, dw) Zulkowsky starch in 0.1 M NaAC pH 5.0, 10 mM $CaCl_2$. Temperature 60 °C. A. niger glucoamylase, 22.5 units (Sigma)/g of starch. | | |
| | Relative amount of glucose (%) | |
| αAP | Incubation time | |
| kUp/g | 48 h | 96 h |
| 0 | 88.1 | 90.5 |
| 2 | 92.3 | 95.0 |
| 10 | 94.9 | 93.3 |

Table 3.

| The effect of α-amylase-pullulanase (αAP) on maltose formation by maltogenic α-amylase. | | |
|---|---|---|
| Substrate, 25 % (w/w, dw) Zulkowsky starch in 0.1 M NaAC pH 5.5, 10 mM $CaCl_2$. Temperature 70° C. B. stearothermophilus maltogenic α-amylase (Maltogenase, Novo), 7.5 MANU g of starch. | | |
| | Relative amount of maltose + maltotriose (%) | |
| αAP | Incubation time | |
| kUP/g | 48 h | 96 h |
| 0 | 60.2 | 60.0 |
| 2 | 61.9 | 63.0 |
| 10 | 63.0 | 63.9 |

## Claims

1. A nucleic acid having a sequence comprising:
(a) the sequence of Figs. 5A to 5F;
(b) a sequence encoding a protein having the amino acid sequence of Figs. 5A to 5F;
(c) a sequence as in (a) and/or (b) with a multiple base substitution, deletion, insertion or inversion; or
(d) a portion of a sequence in (a), (b) or (c).

2. A nucleic acid as claimed in claim 1 which encodes for an enzyme with pullulanase and alpha-amylase activities, and is obtainable from a microorganism of the genus Clostridium.

3. A nucleic acid as claimed in claim or 2 comprising a sequence or portion thereof encoding for an enzyme with pullulanase and alpha-amylase activities, and is obtainable from Clostridium thermohydrosulphuricum.

4. A recombanant DNA molecule comprising at least one DNA fragment encoding an enzyme having both pullulanase and alpha-amylase activities, the fragment comprising a DNA sequence as claimed in any of claims 1 to 3.

5. A recombinant DNA molecule as claimed in claim 4 comprising at least one DNA fragment derivable from a microorganism of the genus Clostridium or portions thereof encoding for an enzyme with pullulanase and alpha-amylase activities.

6. A protein encoded by a nucleic acid sequence as claimed in any of claims 1 to 3 or as shown in Figs. 5A to 5F, or a fragment thereof.

7. An enzyme having alpha-amylase and pullulanase activities and comprising the amino acid sequence of Figs 5A to 5F or a portion thereof possessing said activities.

8. An enzyme as claimed in claim 7 comprising an amino acid sequence coded from a DNA fragment derivable from a microorganism of the genus Clostridium, such as from Clostridium thermohydrosulphuricum (e.g. DSM 3783).

9. A method for isolating a DNA fragment coding for an enzyme with pullulanase and alpha-amylase activity, the method comprising:
(a) digesting chromosomal DNA from a Clostridium strain, such as chromosomal DNA derivable from Clostridium thermohydrosulphuricum (DSM 3783);
(b) inserting resultant DNA fragments into a suitable vector;
(c) infecting a suitable host with the vector; and
(d) screening immunologically (such as with a rabbit alpha-amylase pullulanase antiserum), enzymatically (such as with a starch containing layer stained with iodine), or by a hybridization method (such as hybridizing with a synthetic DNA probe comprising a probe for the enzyme or a satellite protein).

10. A process for the preparation of an enzyme having pullulanase and alpha-amylase activity, the process comprising:

(a) selecting a DNA fragment encoding for the enzyme;

(b) inserting the fragment into a suitable vector to obtain a recombinant DNA molecule;

(c) introducing the recombinant DNA molecule into a suitable host, such as Escherichia coli, to obtain a recombinant clone;

(d) cultivating the recombinant clone in a suitable medium;

(e) recovering the enzyme after cultivation, such as after disintegrating the cells.

11. A vector comprising a nucleic acid sequence as claimed in any of claims 1 to 3.

12. A host transfected by a vector as claimed in claim 11.

13. A process for hydrolysing starch, amylose or pullulan, the process comprising contacting starch, amylose and/or pullulan with a protein as claimed in claim 6 or an enzyme preparable by a process as claimed in claim 10, alone or with a glucogenic or maltogenic enzyme.

14. A recombinant protein having alpha-amylase and pullulanase activity.

Claims for the following Contracting State: ES

1. A process for the preparation of a nucleic acid having a sequence comprising:

(a) the sequence of Figs. 5A to 5F;

(b) a sequence encoding a protein having the amino acid sequence of Figs. 5A to 5F;

(c) a sequence as in (a) and/or (b) with a multiple base substitution, deletion, insertion or inversion; or

(d) a portion of a sequence in (a), (b) or (c); the process comprising coupling successive nucleotides together.

2. A process as claimed in claim 1 wherein the nucleic acid encodes for an enzyme with pullulanase and alpha-amylase activities, and is obtainable from a microorganism of the genus Clostridium.

3. A process as claimed in claim 1 or 2 wherein the nucleic acid comprises a sequence or portion thereof encoding for an enzyme with pullulanase and alpha-amylase activities, and is obtainable from Clostridium thermohydrosulphuricum.

4. A process for the preparation of a recombanant DNA molecule comprising at least one DNA fragment encoding an enzyme having both pullulanase and alpha-amylase activities, the fragment comprising a DNA sequence as claimed in any of claims 1 to 3, the process comprising coupling successive nucleotides together.

5. A process as claimed in claim 4 wherein the molecule comprises at least one DNA fragment derivable from a microorganism of the genus Clostridium or portions thereof encoding for an enzyme with pullulanase and alpha-amylase activities.

6. A process for the preparation of a protein encoded by a nucleic acid sequence as claimed in any of claims 1 to 3 or as shown in Figs. 5A to 5F, or a fragment thereof, the process comprising coupling successive amino acids together.

7. A process for the preparation of an enzyme having alpha-amylase and pullulanase activities and comprising the amino acid sequence of Figs 5A to 5F or a portion thereof possessing said activities, the process comprising coupling successive amino acids together.

8. A process as claimed in claim 7 wherein the enzyme comprises an amino acid sequence coded from a DNA fragment derivable from a microorganism of the genus Clostridium, such as from Clostridium thermohydrosulphuricum (e.g. DSM 3783).

9. A method for isolating a DNA fragment coding for an enzyme with pullulanase and alpha-amylase activity, the method comprising:

(a) digesting chromosomal DNA from a Clostridium strain, such as chromosomal DNA derivable from Clostridium thermohydrosulohuricum (DSM 3783);

(b) inserting resultant DNA fragments into a suitable vector;

(c) infecting a suitable host with the vector; and

(d) screening immunologically (such as with a rabbit alpha-amylase pullulanase antiserum), enzymatically (such as with a starch containing layer stained with iodine), or by a hybridization method (such as hybridizing with a synthetic DNA probe comprising a probe for the enzyme or a satellite protein).

10. A process for the preparation of an enzyme having pullulanase and alpha-amylase activity, the process comprising:

(a) selecting a DNA fragment encoding for the enzyme;

(b) inserting the fragment into a suitable vector to obtain a recombinant DNA molecule;

(c) introducing the recombinant DNA molecule into a suitable host, such as Escherichia coli, to obtain a recombinant clone;

(d) cultivating the recombinant clone in a suitable medium;

(e) recovering the enzyme after cultivation, such as after disintegrating the cells.

11. A process for the preparation of a vector the process comprising inserting a nucleic acid sequence as claimed in any of claims 1 to 3 into another nucleic acid sequence.

12. A process comprising transfecting a host by a vector as claimed in claim 11.

13. A process for hydrolysing starch, amylose or pullulan, the process comprising contacting starch, amylose and/or pullulan with a protein as claimed in claim 6 or an enzyme preparable by a process as claimed in claim 10, alone or with a glucogenic or maltogenic enzyme.

14. A process for the preparation of a recombinant protein having alpha-amylase and pullulanase activity the process comprising coupling successive amino acids together.

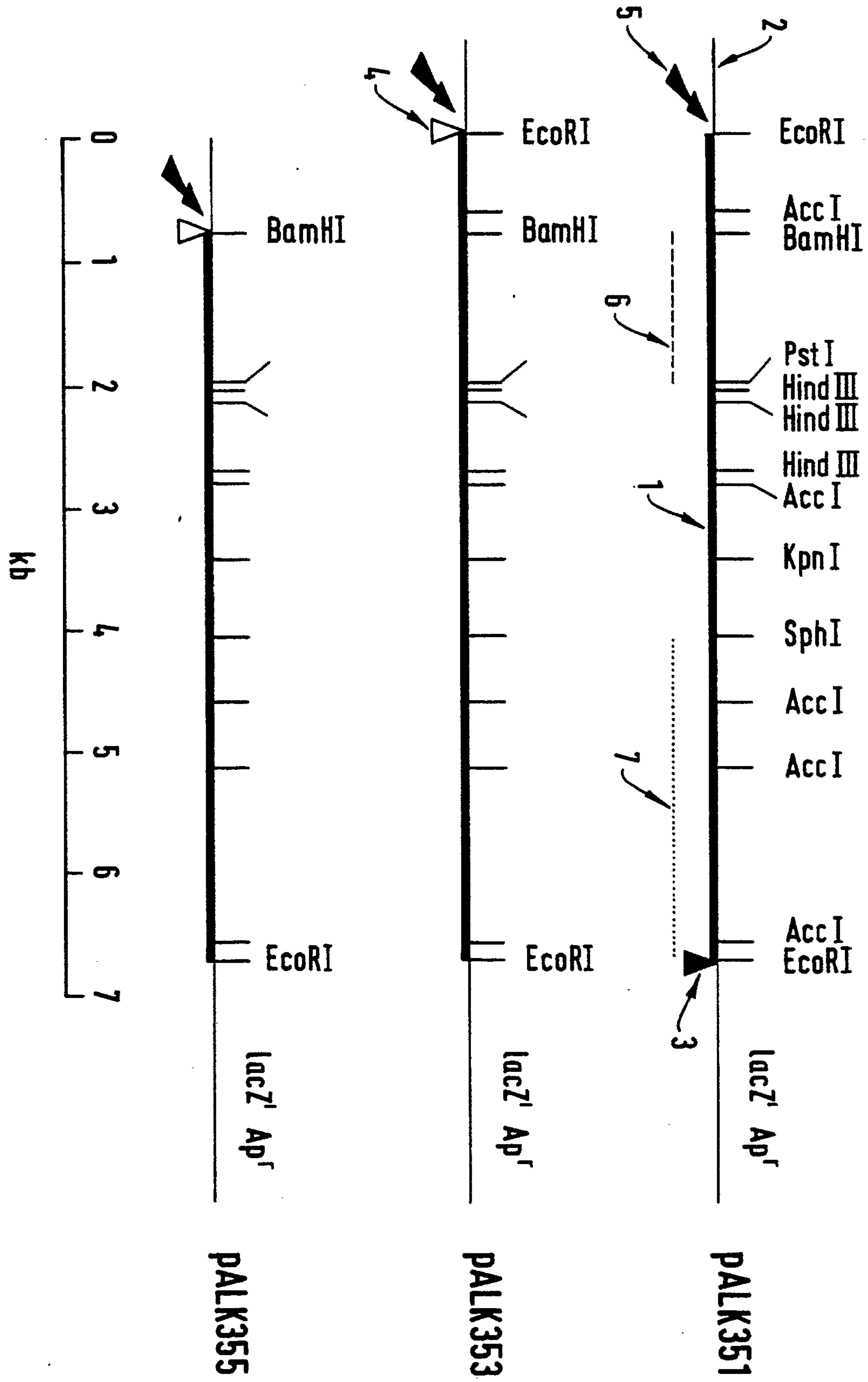

kb
0
1
2
3
4
5
6
7
1
2
3
4
5
6
7
EcoRI
AccI
BamHI
PstI
HindIII
HindIII
HindIII
AccI
KpnI
SphI
AccI
AccI
AccI
EcoRI
lacZ'
Ap^r
pALK351
pALK353
pALK355


Fig. 1

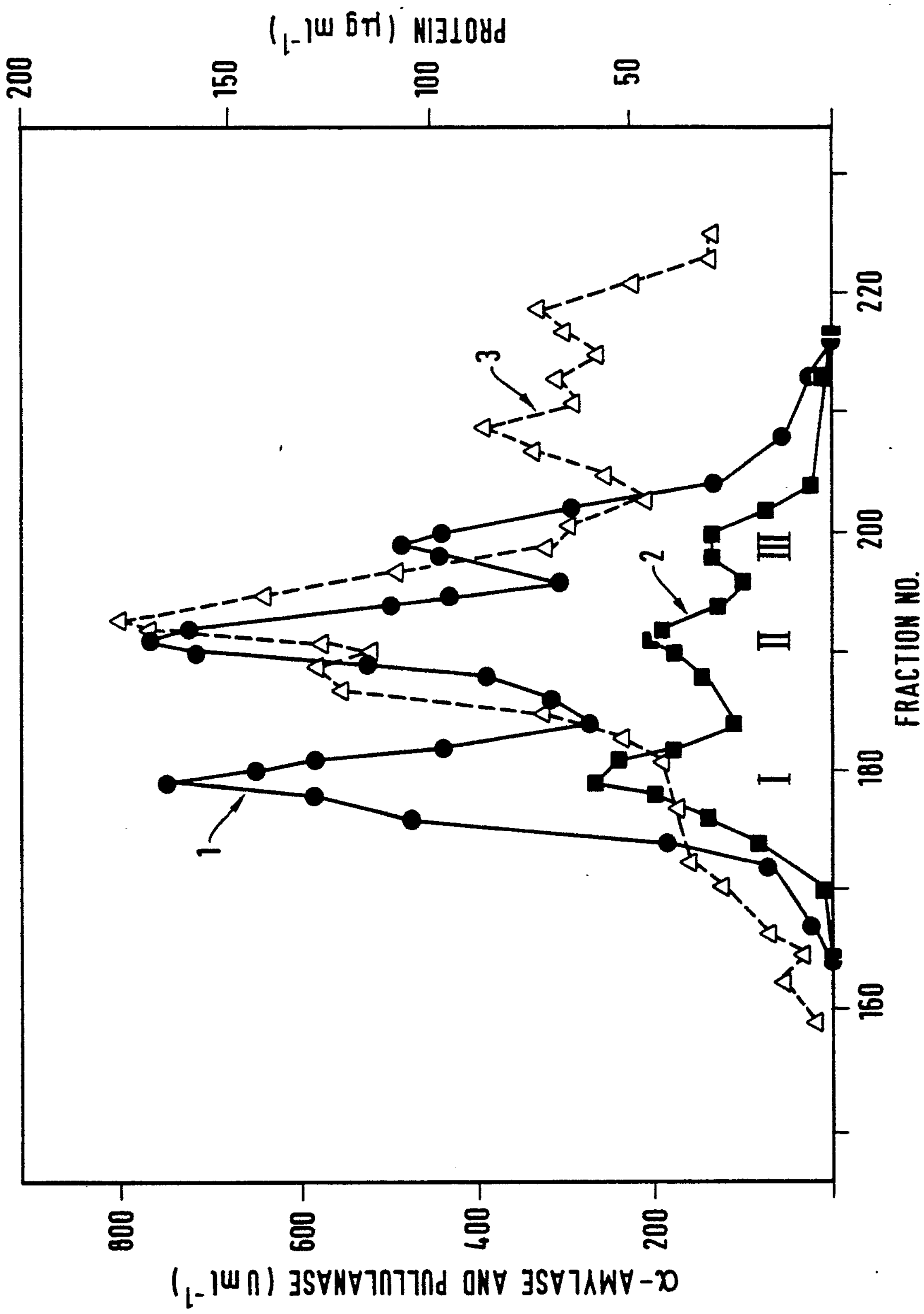
PROTEIN (μg ml-1)
200
150
100
50
α-AMYLASE AND PULLULANASE (U ml-1)
800
600
400
200
FRACTION NO.
160
180
200
220
1
2
3
I
II
III

Fig. 2

Fig. 3

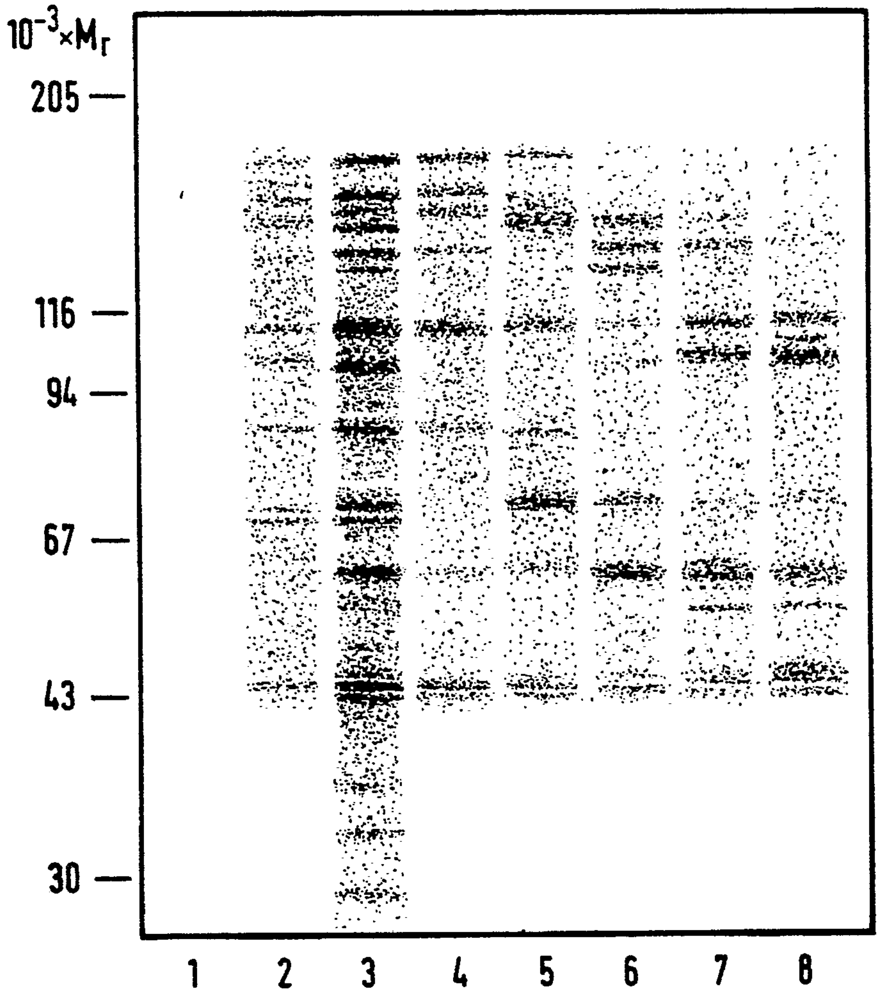

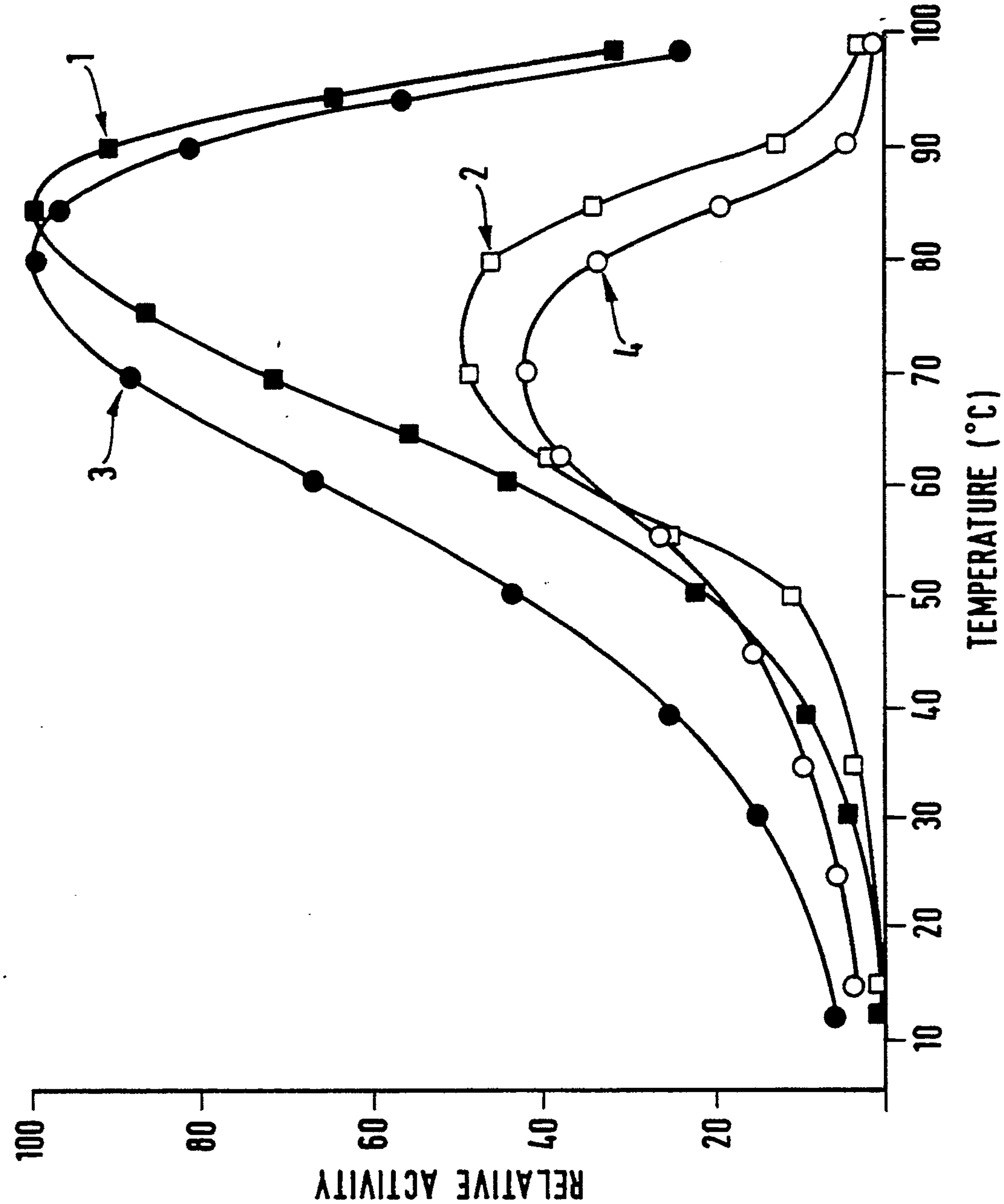
1
2
3
4
100
80
60
40
20
RELATIVE ACTIVITY
10
20
30
40
50
60
70
80
90
100
TEMPERATURE (°C)

Fig. 4

# Fig. 5A

TTTAGCAGAAAAAGCAGATAAAA 23

TGGAGATAACACAAAGGTTAAAGCAGATATAATTTATAGGGCTGCTTTATAGTAGCCCT 82

ATAAATTTACATATAGTGTATGTATTAACAACATATTGACAACGAAAAAACTATAATTT 141

ATAATAGACATGAGGGAAAACGATTGCGCAAAAATTTGCACAAATAAAATGTGTTTTTA 200

TCACATTTATCTTTTTGCAATTTGTGAAAGCGATTGCCTAAAAGAAATGGGGGTGGGAT 259

TGGGAATTAATTTTTTTAAAAACATTTAAGTAAATATATGGATAAAAGGGGGATTATAG 318

GTG TTT AAA AGG AGA GCT TTA GGA TTT TTG CTG GCT TTT CTT TTA 363
-31 Met Phe Lys Arg Arg Ala Leu Gly Phe Leu Leu Ala Phe Leu Leu

GTT TTT ACA GCA GTA TTT GGC TCA ATG CCT ATG GAA TTT GCA AAG 408
-16 Val Phe Thr Ala Val Phe Gly Ser Met Pro Met Glu Phe Ala Lys

GCT GAG ACA GAT ACA GCG CCA GCG ATA GCC AAT GTT GTT GGC AAT 453
-1 Ala Glu Thr Asp Thr Ala Pro Ala Ile Ala Asn Val Val Gly Asn

TTT CAA TCC AAA CTT GGA GAT TCT GAC TGG AAT ATA AAC AGT GAC 498
15 Phe Gln Ser Lys Leu Gly Asp Ser Asp Trp Asn Ile Asn Ser Asp

AAA ACG ATA ATG ACA TAT AAA GGT AAT GGC TTT TAT GAA TTT ACT 543
30 Lys Thr Ile Met Thr Tyr Lys Gly Asn Gly Phe Tyr Glu Phe Thr

ACC CCA GTT GCG TTA CCT GCA GGT GAT TAT GAG TAT AAA GTT GCT 588
45 Thr Pro Val Ala Leu Pro Ala Gly Asp Tyr Glu Tyr Lys Val Ala

CTT AAT CAT TCA TGG GAA GGT GGA GGA GTT CCT TCA CAA GGT AAT 633
60 Leu Asn His Ser Trp Glu Gly Gly Gly Val Pro Ser Gln Gly Asn

TTA AGC TTT CAT CTT GAT TCA GAT TCT GTA GTG ACT TTT TAT TAC 678
75 Leu Ser Phe His Leu Asp Ser Asp Ser Val Val Thr Phe Tyr Tyr

AAT TAC AAC ACT TCA AGT ATT ACC GAT TCC ACA AAA TAT ACA CCA 723
90 Asn Tyr Asn Thr Ser Ser Ile Thr Asp Ser Thr Lys Tyr Thr Pro

ATT CCC GAA GAC AAG CTT CCA AGA CTT GTA GGG ACT ATA CAG CCT 768
105 Ile Pro Glu Asp Lys Leu Pro Arg Leu Val Gly Thr Ile Gln Pro

GCA ATA GGA GCA GGT GAT GAT TGG AAA CCT GAA ACA TCG ACT GCT 813
120 Ala Ile Gly Ala Gly Asp Asp Trp Lys Pro Glu Thr Ser Thr Ala

ATA ATG AGA GAC TAT AAG TTT AAT AAT GTT TAC GAA TAC ACT GCA 858
135 Ile Met Arg Asp Tyr Lys Phe Asn Asn Val Tyr Glu Tyr Thr Ala

AAT GTT CCA AAA GGG AAT TAT GAG TTT AAA GTG ACT TTA GGG CCC 903
150 Asn Val Pro Lys Gly Asn Tyr Glu Phe Lys Val Thr Leu Gly Pro

TCA TGG GAT ATA AAT TAT GGC TTA AAT GGT GAA CAA AAT GGG CCA 948
165 Ser Trp Asp Ile Asn Tyr Gly Leu Asn Gly Glu Gln Asn Gly Pro

AAT ATT CCT TTG AAT GTA GCC TAT GAT ACT AAG ATT ACA TTT TAC 993
180 Asn Ile Pro Leu Asn Val Ala Tyr Asp Thr Lys Ile Thr Phe Tyr

**Fig. 5B**

```
     TAT GAT TCG GTT TCA CAT AAT ATA TGG ACA GAT TAT AAT CCA CCT   1038
195  Tyr Asp Ser Val Ser His Asn Ile Trp Thr Asp Tyr Asn Pro Pro

     CTT ACA GGA CCT GAT AAT AAC ATA TAT TAT GAC GAT TTA AGA CAT   1083
210  Leu Thr Gly Pro Asp Asn Asn Ile Tyr Tyr Asp Asp Leu Arg His

     GAC ACC CAT GAC CCA TTC TTC CGC TCG CCT TTC GGT GCA ATA AAA   1128
225  Asp Thr His Asp Pro Phe Phe Arg Ser Pro Phe Gly Ala Ile Lys

     ACG GGA GAT ACT GTG ACA TTA AGA ATA CAA GCA AAA AAT CAT GAC   1173
240  Thr Gly Asp Thr Val Thr Leu Arg Ile Gln Ala Lys Asn His Asp

     ATT GAG TCA GCT AAA ATT TCT TAT TGG GAT GAT ATT AAA AAA ACA   1218
255  Ile Glu Ser Ala Lys Ile Ser Tyr Trp Asp Asp Ile Lys Lys Thr

     AGA ATA GAA GTC CCT ATG TAC AGA ATT GGT CAA AGT CCT GAC GGG   1263
270  Arg Ile Glu Val Pro Met Tyr Arg Ile Gly Gln Ser Pro Asp Gly

     AAA TAT GAA TAC TGG GAA GTT AAG TTA AGC TTT GAC CAT CCC ACA   1308
285  Lys Tyr Glu Tyr Trp Glu Val Lys Leu Ser Phe Asp His Pro Thr

     AGA ATT TGG TAT TAC TTT ATA CTT AAA GAC GGG ACC AAA ACT GCT   1353
300  Arg Ile Trp Tyr Tyr Phe Ile Leu Lys Asp Gly Thr Lys Thr Ala

     TAT TAC GGA GAT AAC GAT GAA CAA TTA GGT GGC GTA GGT AAA GCC   1398
315  Tyr Tyr Gly Asp Asn Asp Glu Gln Leu Gly Gly Val Gly Lys Ala

     ACA GAT ACA GAA AAT AAG GAC TTC GAA CTT ACT GTC TAC GAT AAA   1443
330  Thr Asp Thr Glu Asn Lys Asp Phe Glu Leu Thr Val Tyr Asp Lys

     AAC TTA GAC ACC CCT GAT TGG ATG AAA GGG TCA GTA ATG TAT CAA   1488
345  Asn Leu Asp Thr Pro Asp Trp Met Lys Gly Ser Val Met Tyr Gln

     ATA TTC CCT GAT AGG TTC TTT AAT GGG GAT TCT TCA AAT GAC CAT   1533
360  Ile Phe Pro Asp Arg Phe Phe Asn Gly Asp Ser Ser Asn Asp His

     CTA AAG AAA TAC AGC AGA GGT TTT GAT CCT GTT GAA TAT CAT AGC   1578
375  Leu Lys Lys Tyr Ser Arg Gly Phe Asp Pro Val Glu Tyr His Ser

     AAC TGG TAC GAG CTT CCA GAT AAT CCG AAT GAT AAA AAT AAA CTA   1623
390  Asn Trp Tyr Glu Leu Pro Asp Asn Pro Asn Asp Lys Asn Lys Leu

     GGA TAT ACA GGG GAT GGC ATA TGG TCA AAT GAC TTC TTT GGC GGT   1668
405  Gly Tyr Thr Gly Asp Gly Ile Trp Ser Asn Asp Phe Phe Gly Gly

     GAT TTA AAA GGT ATA GAT GAT AAA TTG GAT TAT TTA AAA AGC CTT   1713
420  Asp Leu Lys Gly Ile Asp Asp Lys Leu Asp Tyr Leu Lys Ser Leu

     GGA ATA TCA GTC ATT TAT CTC AAT CCA ATT TTT CAA TCA CCT TCT   1758
435  Gly Ile Ser Val Ile Tyr Leu Asn Pro Ile Phe Gln Ser Pro Ser

     AAT CAC AGG TAC GAT ACA ACC GAT TAC ACA AAA ATA GAC GAA CTA   1803
450  Asn His Arg Tyr Asp Thr Thr Asp Tyr Thr Lys Ile Asp Glu Leu

     TTA GGA GAT TTA TCT ACC TTT AAG AAG CTC ATG GAG GAT GCC CAT   1848
465  Leu Gly Asp Leu Ser Thr Phe Lys Lys Leu Met Glu Asp Ala His
```

## Fig. 5C

```
     GCA AAA GGG ATT AAG GTA ATA CTT GAT GGC GTC TTC AAT CAT ACA  1893
480  Ala Lys Gly Ile Lys Val Ile Leu Asp Gly Val Phe Asn His Thr

     AGT GAT GAC AGC ATT TAT TTT GAT AGA TAT GGT AAA TAC TTA AAC  1938
495  Ser Asp Asp Ser Ile Tyr Phe Asp Arg Tyr Gly Lys Tyr Leu Asn

     ACG GGA GTT TTA GGT GCT TAT CAA GCA TGG AAA CAG GGA GAT CAG  1983
510  Thr Gly Val Leu Gly Ala Tyr Gln Ala Trp Lys Gln Gly Asp Gln

     TCA AAG TCT CCA TAT GGT GAC TGG TAT GAG ATA AAG CCT GAC GGT  2028
525  Ser Lys Ser Pro Tyr Gly Asp Trp Tyr Glu Ile Lys Pro Asp Gly

     ACC TAT GAA GGA TGG TGG GGA TTT GAC AGT TTA CCT GTG ATA AGG  2073
540  Thr Tyr Glu Gly Trp Trp Gly Phe Asp Ser Leu Pro Val Ile Arg

     CAG ATA AAC GGC AGT GAA TAT AAT GTC AAA AGT TGG GCG GAT TTT  2118
555  Gln Ile Asn Gly Ser Glu Tyr Asn Val Lys Ser Trp Ala Asp Phe

     ATT ATA AAT AAT CCT AAT GCA ATA TCT AAG TAT TGG TTA AAT CCT  2163
570  Ile Ile Asn Asn Pro Asn Ala Ile Ser Lys Tyr Trp Leu Asn Pro

     GAT GGG GAC AAA AAT GTA GGT GCA GAT GGT TGG AGA TTA GAT GTT  2208
585  Asp Gly Asp Lys Asn Val Gly Ala Asp Gly Trp Arg Leu Asp Val

     GCG AAT GAA GTT GCT CAC GAT TTT TGG GTT CAT TTT AGA GGT GCA  2253
600  Ala Asn Glu Val Ala His Asp Phe Trp Val His Phe Arg Gly Ala

     ATT AAT ACT GTG AAA CCG AAT GCT CCG ATG GTT GCA GAA AAC TGG  2298
615  Ile Asn Thr Val Lys Pro Asn Ala Pro Met Val Ala Glu Asn Trp

     AAT GAC GCT TCA CTG GAT CTG CTT GGA GAT TCT TTT AAC TCT GTT  2343
630  Asn Asp Ala Ser Leu Asp Leu Leu Gly Asp Ser Phe Asn Ser Val

     ATG AAT TAT CTC TTT AGA AAT GCA GTA ATT GAC TTT ATA TTG GAT  2388
645  Met Asn Tyr Leu Phe Arg Asn Ala Val Ile Asp Phe Ile Leu Asp

     AAA TCA TTT GAT GAC GGA AAT GTG GTT CAC AAT CCT ATA GAT GCA  2433
660  Lys Ser Phe Asp Asp Gly Asn Val Val His Asn Pro Ile Asp Ala

     GCA AAA CTC GAC CAA AGG CTT ATG AGC ATA TAT GAG AGA TAT CCT  2478
675  Ala Lys Leu Asp Gln Arg Leu Met Ser Ile Tyr Glu Arg Tyr Pro

     CTT CCA GTG TTT TAT TCT ACT ATG AAT CTT TTA GGT TCT CAT GAC  2523
690  Leu Pro Val Phe Tyr Ser Thr Met Asn Leu Leu Gly Ser His Asp

     ACC ATG AGG ATA TTG ACA GTA TTT GGA TAC AAC TCT GCG GAT GAA  2568
705  Thr Met Arg Ile Leu Thr Val Phe Gly Tyr Asn Ser Ala Asp Glu

     AAT CAA AAT TCT CAA GCA GCA AAA GAC CTT GCG GTT AAA AGA CTT  2613
720  Asn Gln Asn Ser Gln Ala Ala Lys Asp Leu Ala Val Lys Arg Leu

     AAA CTT GCT GCA ATA CTG CAA ATG GGT TAT CCA GGC ATG CCT TCT  2658
735  Lys Leu Ala Ala Ile Leu Gln Met Gly Tyr Pro Gly Met Pro Ser

     ATT TAT TAC GGA GAT GAA GCA GGA CAA TCT GGC GGA AAA GAC CCG  2703
750  Ile Tyr Tyr Gly Asp Glu Ala Gly Gln Ser Gly Gly Lys Asp Pro
```

## Fig. 5D

GAC AAC AGA AGG ACA TTC CCT TGG GGA AGA GAA GAT ACT GAT TTG 2748
765 Asp Asn Arg Arg Thr Phe Pro Trp Gly Arg Glu Asp Thr Asp Leu

CAA ACT TTT TTC AAG AAA GTT GTA AAT ATA AGA AAT GAG AAT CAA 2793
780 Gln Thr Phe Phe Lys Lys Val Val Asn Ile Arg Asn Glu Asn Gln

GTT TTG AAA ACA GGA GAC CTT GAG ACA CTT TAT GCA AAT GGC GAT 2838
795 Val Leu Lys Thr Gly Asp Leu Glu Thr Leu Tyr Ala Asn Gly Asp

GTT TAT GCC TTT GGA AGA AGA ATC ATA AAT GGA AAA GAT ACT TTT 2883
810 Val Tyr Ala Phe Gly Arg Arg Ile Ile Asn Gly Lys Asp Thr Phe

GGG AAA TCG TAT CCT GAC AGT GTA GCT ATT GTC GTA ATA AAT AAA 2928
825 Gly Lys Ser Tyr Pro Asp Ser Val Ala Ile Val Val Ile Asn Lys

GGT GAC GCA AAA CAG GTT TCT ATA GAT ACC ACT AAA TTT ATA AGA 2973
840 Gly Asp Ala Lys Gln Val Ser Ile Asp Thr Thr Lys Phe Ile Arg

GAT GGA GTT GCT TTT ACA GAT GCC TTA AGT GGC AAG ACA TAC ACG 3018
855 Asp Gly Val Ala Phe Thr Asp Ala Leu Ser Gly Lys Thr Tyr Thr

GTT CAG GAT GGT AAA ATT GTT GTA GAA GTT GGA TCA ATG GAT GGA 3063
870 Val Gln Asp Gly Lys Ile Val Val Glu Val Gly Ser Met Asp Gly

GCT ATA CTT ATA TCA GAT ACA GGA CAA AAT TTG ACA GCA CCT CAG 3108
885 Ala Ile Leu Ile Ser Asp Thr Gly Gln Asn Leu Thr Ala Pro Gln

CCA ATA ACA GAC CTT AAA GCA GTC TCA GGG AAT GGC AAA GTA GAC 3153
900 Pro Ile Thr Asp Leu Lys Ala Val Ser Gly Asn Gly Lys Val Asp

CTT TCC TGG AGT GTA GTA GAT AAA GCA GTA AGC TAT AAT ATT TAC 3198
915 Leu Ser Trp Ser Val Val Asp Lys Ala Val Ser Tyr Asn Ile Tyr

CGT TCT ACA GTT AAA GGT GGG TTA TAT GAA AAA ATA GCT TCA AAT 3243
930 Arg Ser Thr Val Lys Gly Gly Leu Tyr Glu Lys Ile Ala Ser Asn

GTT ACG CAA ATT ACC TAT ACT GAT ACA GAG GTT ACC AAT GGT CTA 3288
945 Val Thr Gln Ile Thr Tyr Thr Asp Thr Glu Val Thr Asn Gly Leu

AAG TAT GTG TAT GCT GTA ACC GCT GTA GAT AAT GAT GGA AAT GAG 3333
960 Lys Tyr Val Tyr Ala Val Thr Ala Val Asp Asn Asp Gly Asn Glu

AGT GCT TTA AGC AAT GAA GTT GAG GCA TAT CCA GCA TTT CCT ATT 3378
975 Ser Ala Leu Ser Asn Glu Val Glu Ala Tyr Pro Ala Phe Pro Ile

GGC TGG GCG GGA AAT ATG AAT CAA GTA AAT ACA CAT GTA ATA GGT 3423
990 Gly Trp Ala Gly Asn Met Asn Gln Val Asn Thr His Val Ile Gly

GTA AAT AAT CCA GTT GAA GTG TAT GCT GAA GTG TGG GCT CAA GGG 3468
1005 Val Asn Asn Pro Val Glu Val Tyr Ala Glu Val Trp Ala Gln Gly

CTT ACA GAT AAA CCT GGC CAA GGA GAA AAT ATG ATT GCC CAG TTA 3513
1020 Leu Thr Asp Lys Pro Gly Gln Gly Glu Asn Met Ile Ala Gln Leu

GGA TAT AGG TAT ATT GGA GAT ACT GTA GGA GAT GCA GTT TAC AAT 3558
1035 Gly Tyr Arg Tyr Ile Gly Asp Thr Val Gly Asp Ala Val Tyr Asn

**Fig. 5E**

```
      GCC GTG TAC AAT AAG GTA GAA GGT GTT GAA ATA AGT AAG GAC TGG   3603
1050  Ala Val Tyr Asn Lys Val Glu Gly Val Glu Ile Ser Lys Asp Trp

      ACA TGG GTT GAT GCA CAA TAT GTA GGT GAT TCT GGA AAT AAT GAT   3648
1065  Thr Trp Val Asp Ala Gln Tyr Val Gly Asp Ser Gly Asn Asn Asp

      AAA TAC ATG GCT AAA TTT GTA CCT GAT ATG GTA GGC ACA TGG GAA   3693
1080  Lys Tyr Met Ala Lys Phe Val Pro Asp Met Val Gly Thr Trp Glu

      TAC ATT ATG AGA TTT TCT TCT AAC CAA GGC CAT GAT TGG ACG TAT   3738
1095  Tyr Ile Met Arg Phe Ser Ser Asn Gln Gly His Asp Trp Thr Tyr

      ACA AAA GGA CCA GAT GGG AAA ACA GAT GAA GCA AAA CAG TTT ACT   3783
1110  Thr Lys Gly Pro Asp Gly Lys Thr Asp Glu Ala Lys Gln Phe Thr

      GTC GTG CCA TCA AAT GAT GTA GAA ACA CCT ACA GCT CCA GTC TTA   3828
1125  Val Val Pro Ser Asn Asp Val Glu Thr Pro Thr Ala Pro Val Leu

      CAA CAA CCA GGA ATT GAA TCC TCA AGA GTT ACA CTT AAC TGG AGT   3873
1140  Gln Gln Pro Gly Ile Glu Ser Ser Arg Val Thr Leu Asn Trp Ser

      CCG TCA GCT GAT GAT GTT GCT ATT TTC GGC TAC GAA ATC TAT AAG   3918
1155  Pro Ser Ala Asp Asp Val Ala Ile Phe Gly Tyr Glu Ile Tyr Lys

      TCT TCA AGT GAG ACA GGA CCA TTT ATA AAG ATT GCA ACT GTG TCT   3963
1170  Ser Ser Ser Glu Thr Gly Pro Phe Ile Lys Ile Ala Thr Val Ser

      GAC AGT GTG TAT AAC TAC GTA GAT ACA GAT GTA GTA AAT GGC AAT   4008
1185  Asp Ser Val Tyr Asn Tyr Val Asp Thr Asp Val Val Asn Gly Asn

      GTG TAT TAT TAT AAA GTT GTG GCA GTT GAT ACC TCC TAT AAT AGG   4053
1200  Val Tyr Tyr Tyr Lys Val Val Ala Val Asp Thr Ser Tyr Asn Arg

      ACA GCA TCA AAT ACA GTA AAA GCG ACA CCT GAT ATA ATA CCT ATA   4098
1215  Thr Ala Ser Asn Thr Val Lys Ala Thr Pro Asp Ile Ile Pro Ile

      AAA GTG ACA TTT AAT GTC ACA ATA CCA GAT TAT ACT CCT GAT GAT   4143
1230  Lys Val Thr Phe Asn Val Thr Ile Pro Asp Tyr Thr Pro Asp Asp

      GGT GTA AAT ATT GCT GGA AAC TTC CCG GAT GCT TTC TGG AAT CCA   4188
1245  Gly Val Asn Ile Ala Gly Asn Phe Pro Asp Ala Phe Trp Asn Pro

      AAT GCT AAT CAA ATG ACA AAA GCT GGT TCT AAC ACC TAT AGT ATT   4233
1260  Asn Ala Asn Gln Met Thr Lys Ala Gly Ser Asn Thr Tyr Ser Ile

      ACA CTT ACT TTA AAT GAG GGT ACA CAA ATT GAA TAT AAA TAT GCA   4278
1275  Thr Leu Thr Leu Asn Glu Gly Thr Gln Ile Glu Tyr Lys Tyr Ala

      AGG GGG AGC TGG GAT AAA GTA GAA AAA GGT GAA TAC GGC AAT GAG   4323
1290  Arg Gly Ser Trp Asp Lys Val Glu Lys Gly Glu Tyr Gly Asn Glu

      ATT GAC AAC AGA AAA ATA ACT GTT GTC AAT CAG GGT TCA AAT ACA   4368
1305  Ile Asp Asn Arg Lys Ile Thr Val Val Asn Gln Gly Ser Asn Thr

      ATG GTT GTA AAT GAT ACA GTG CAA AGA TGG AGA GAT GTG CCA ATA   4413
1320  Met Val Val Asn Asp Thr Val Gln Arg Trp Arg Asp Val Pro Ile
```

**Fig. 5F**

```
      TAT ATC TAT TCT CCA AAA GAC AAG ACT ATT GTT GAT GCA AAT ACG   4458
1335  Tyr Ile Tyr Ser Pro Lys Asp Lys Thr Ile Val Asp Ala Asn Thr

      AGT GAA ATA GAG ATT AAA GGC AAT ACT TAC AAA GGT GCG AAA GTA   4503
1350  Ser Glu Ile Glu Ile Lys Gly Asn Thr Tyr Lys Gly Ala Lys Val

      ACT ATA AAT GAT GAA TCT TTT GTG CAA CAA GAA AAT GGA GTA TTT   4548
1365  Thr Ile Asn Asp Glu Ser Phe Val Gln Gln Glu Asn Gly Val Phe

      ACA AAA GTA GTT CCC TTG GAA TAT GGT GTA AAT ACT ATC AAA ATA   4593
1380  Thr Lys Val Val Pro Leu Glu Tyr Gly Val Asn Thr Ile Lys Ile

      CAT GTA GAG CCA AGT GGT GAC AAG AAT AAT GAA CTT ACA AAA GAT   4638
1395  His Val Glu Pro Ser Gly Asp Lys Asn Asn Glu Leu Thr Lys Asp

      ATA ACA ATA ACT GTT ACA AGA GAG AAG CCG GCC AGG AGA CAG AAC   4683
1410  Ile Thr Ile Thr Val Thr Arg Glu Lys Pro Ala Arg Arg Gln Asn

      CTA CTC CTA CTC CAC CAA CAG AAA CAA CAA AAC CAT CTC AAG AAG   4728
1425  Leu Leu Leu Leu His Gln Gln Lys Gln Gln Asn His Leu Lys Lys

      TAT CAC AAG GCA AAA TAGTTGTAGAAAATAACACAACAACACTTACAATAGACG   4773
1440  Tyr His Lys Ala Lys End   End                           End

      AAAACAAAGTAGCAAAAGAACATAAAAGACACTTCAAAGAAAGAAATACGATTTGAC     4839
1455            End
```

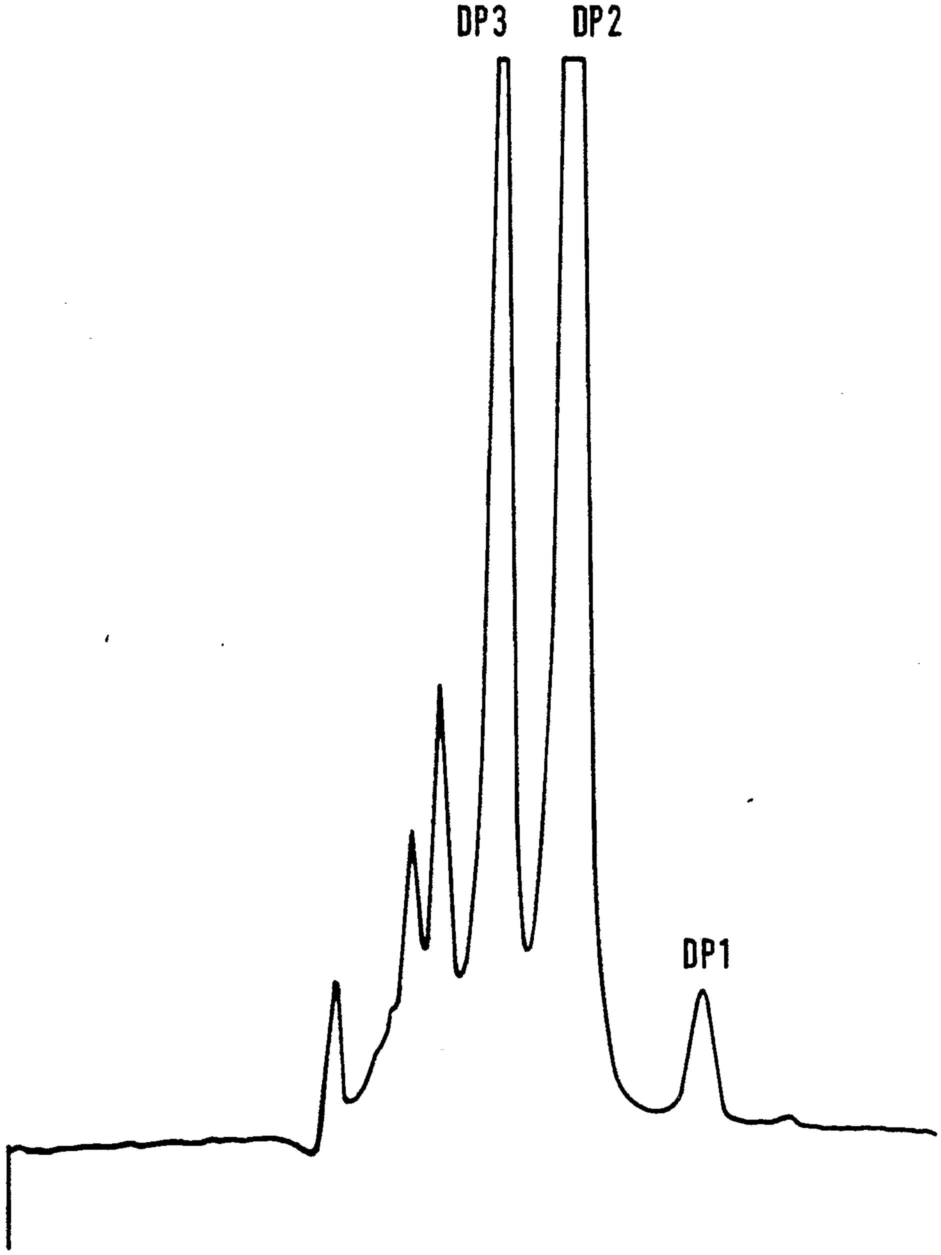


HYDROLYSIS OF 25% (w/w, dw) CORN STARCH
BY α-AMYLASE-PULLULANASE.
INCUBATION 96h AT 70°C.
ENZYME, 100 $U_{p}g^{-1}$; 0.1M NaAc pH 5.5,
10 mM $CaCl_2$.

Fig. 6